# EUROPÄISCHE PATENTSCHRIFT

(11) **EP 3 583 158 B1**
(45) Veröffentlichungstag und Bekanntmachung des Hinweises auf die Patenterteilung: **05.08.2020**
(21) Anmeldenummer: 17732813.5
(22) Anmeldetag: 12.06.2017
(51) Int. Cl.: C08G 77/50, C08L 83/04

(54) **VERFAHREN ZUR HERSTELLUNG VON ORGANOPOLYSILOXANGELEN**
PROCESS FOR PREPARING ORGANOPOLYSILOXANE GELS
PROCÉDÉ DE FABRICATION DE GELS D'ORGANOPOLYSILOXANE

(43) Veröffentlichungstag der Anmeldung: 25.12.2019
(73) Patentinhaber: Wacker Chemie AG, 81737 München (DE)
(72) Erfinder: SANDMEYER, Frank, 84508 Burgkirchen (DE); SCHOSSER, Christoph, 84489 Burghausen (DE)
(74) Vertreter: Deffner-Lehner, Maria
(86) Internationale Anmeldenummer: PCT/EP2017/064241
(87) Internationale Veröffentlichungsnummer: WO 2018/228657

(56) Entgegenhaltungen:
- DE-A1-102012 206 209

## Beschreibung

Die Erfindung betrifft ein Verfahren zur Herstellung von Organopolysiloxangelen sowie deren Verwendung in kosmetischen Zusammensetzungen.

Organopolysiloxangele können durch Vernetzung eines ungesättigten Organopolysiloxanharzes mit einem Si-H-haltigen Organopolysiloxan, im Weiteren auch Si-H-funktioneller Vernetzer genannt, in Gegenwart eines Verdünnungsmittels hergestellt werden.

Vernetzungen sind Verbindungen von Polymerketten in einem dreidimensionalen Netzwerk. Sie können als langkettige Verzweigungen betrachtet werden, die so zahlreich sind, dass ein kontinuierliches unlösliches Netzwerk oder Gel gebildet wird.

Organopolysiloxannetzwerke werden häufig über platinkatalysierte Hydrosilylierungsreaktionen hergestellt. Dabei werden häufig ein Si-H-haltiges Organopolysiloxan und ein Vinyl-funktionelles Organopolysiloxan miteinander zur Reaktion gebracht. Eine wesentliche Voraussetzung für den Aufbau eines 3-dimensionalen Netzwerkes ist dabei, dass mindestens eine der beiden Komponenten, das Si-H-haltige Organopolysiloxan oder das Vinyl-funktionelle Organopolysiloxan, in der mittleren Zusammensetzung mehr als zwei Funktionalitäten pro Molekül aufweist.

Die platinkatalysierte Hydrosilylierungsreaktion bietet bei der Ausbildung von Organopolysiloxannetzwerken den Vorteil, dass keine Nebenprodukte gebildet werden, und dass Verknüpfungsstellen und Netzwerkarchitektur eng definiert sind.

Der wichtigste Grund für die Anwendung von Organopolysiloxangelen in kosmetischen Anwendungen sind die dadurch erzielten sensorischen Vorteile, insbesondere die Verbesserung des Hautgefühls von kosmetischen Formulierungen. Darüber hinaus dienen Organopolysiloxangele als Verdickungsmittel in kosmetischen Formulierungen.

US 6,423,322 B1 offenbart Organopolysiloxangele, die durch Hydrosilylierungsreaktion eines speziellen, vinylfunktionellen MQ-Harzes mit einem Si-H-haltigen Organopolysiloxan in Gegenwart eines Verdünnungsmittels und einer kleinen Menge Platin-Hydrosilylierungskatalysator in einem thermischen Prozess, d.h. unter Aufheizen auf eine Temperatur unterhalb des Siedepunktes des verwendeten Verdünnungsmittels leicht hergestellt werden können. Es ist ein allgemeines Kennzeichen solcher Hydrosilylierungsreaktionen, an denen MQ-Harze beteiligt sind, dass sie nur dann in akzeptabler Geschwindigkeit zum gewünschten Endprodukt ablaufen, wenn sie in der Hitze ausgeführt werden. Dabei unterscheiden sich die möglichen Aufheizraten im Labor und im Produktionsmaßstab deutlich voneinander. Im Falle des Wechsels von einem Rührwerk zu einem anderen treten ebensolche Unterschiede dann in der Regel erneut auf, wenn sich die Rührwerke hinsichtlich ihrer Größe, Materialbeschaffenheit oder technischen Auslegung unterscheiden. Es hat sich gezeigt, dass die erreichbaren Viskositäten solcher MQ-harzhaltigen Gele abhängig sind von der Aufheizrate. Je höher die Aufheizrate ist, desto höher ist die erzielbare Viskosität bei einer vorgegebenen Zusammensetzung und einer festen Konzentration an Gel im Verdünnungsmittel. Sowohl die Konzentration an filmbildendem Gel, bzw. Wirkstoff, als auch die Viskosität haben einen deutlichen Einfluss auf das Hautgefühl einer kosmetischen Zubereitung für die Hautpflege.

US 5,654,362 lehrt Silicongele für kosmetische Anwendungen, die durch Hydrosilylierungsreaktionen von alpha-omega Dienen mit terminal und oder kettenständig Si-H-funktionellen linearen Polyorganosiloxanen erhalten werden. Die Hydrosilylierung findet unter Verwendung eines geeigneten Katalysators in einem Lösemittel statt, welches selbst ein Polyorganosiloxan sein kann.

Diesen Verfahren gemeinsam ist, dass die vernetzten Gelstrukturen durch die Verwendung einer Si-H-haltigen und einer vinylfunktionellen Komponente gebildet werden. Die Möglichkeiten der Steuerung der Produkteigenschaft Viskosität sowohl während der Herstellung als auch in der späteren Anwendung sind damit auf die Auswahl der jeweiligen Rohstoffe und der Menge des jeweiligen Verdünnungs- oder Lösemittels beschränkt. Problematisch ist diese Einschränkung in dem Moment, in dem man auf unveränderliche Rahmenbedingungen trifft, wie sie beispielsweise bei Maßstabsvergrößerungen aus dem Labormaßstab in den Produktionsmaßstab auftreten können. Solche Rahmenbedingungen können insbesondere begrenzte Aufheizraten sein.

Durch eine Umstellung der Rezeptur beispielsweise durch den vollständigen Austausch einer Komponente gegen eine andere und Anpassung an die veränderte Ausgangslage, werden sich die Produkteigenschaften in der Regel weitestgehend verändern, so dass eine Maßstabsvergrößerung des Zielproduktes zu einer unlösbaren Aufgabe wird. Vielmehr muss unter erheblichem Aufwand versucht werden, ein performancegleiches Produkt im größeren Maßstab zu entwickeln.

US 2012/0202895 A1 lehrt eine pastöse Zubereitung für Kosmetikanwendungen, erhalten durch die Hydrosilylierung von einem oder mehreren Si-H-haltigen Polyorganosiloxanen mit einem oder mehreren aliphatisch ungesättigten Polyorganosiloxanen in einem bei 25°C flüssigen Öl als Lösemittel. In diesem Fall handelt es sich bei den eingesetzten Polyorganosiloxanen ausschließlich um lineare Polyorganosiloxane, nicht um Harze. Mindestens zwei der verwendeten Polyorganosiloxane aus der Gruppe der Si-H-haltigen und ungesättigten linearen Polyorganosiloxane müssen eine Kettenlänge von mehr als 30 Wiederholungseinheiten aufweisen, damit in der Anwendung kein öliges, schmieriges Hautgefühl auftritt. Die Viskosität der so erhaltenen Polyorganosiloxanpasten ist in allen Beispielen die gleiche, unabhängig von den Kettenlängen der eingesetzten Si-H-haltigen Polyorganosiloxane, d.h. auch unabhängig davon, ob zwei der Si-H-haltigen Polyorganosiloxane eine Kettenlänge von mehr als 30 aufweisen oder nicht. Somit offenbart US 2012/0202895 A1 zwar die Option zur Steuerung der sensorischen Eigenschaften eines Elastomergels, aber keine Möglichkeit zur Steuerung der Viskosität desselben.

DE102012206209 offenbart ein Verfahren zur Herstellung von Organopolysiloxangelen, wobei ungesättigte Organopolysiloxanharze enthaltend vorwiegend M- und Q-Einheiten mit einem Gemisch aus zwei SiH-haltigen Organopolysiloxanen, die unterschiedliche mittlere Kettenlängen aufweisen, in Verdünnungsmitteln umgesetzt werden.

Es bestand die Aufgabe, ein einfaches Verfahren zur Steuerung der Viskosität von Organopolysiloxangelen bereitzustellen, das die Übertragung des Verfahrens in verschiedene Apparaturen, wie bei Maßstabsvergrößerungen, insbesondere die Übertragung des Verfahrens bei veränderten Rahmenbedingungen, wie Verringerung der Heizrate, unter Erhalt der sensorischen Eigenschaften der Organopolysiloxangele mit minimalen Anpassungen der Rezeptur erlaubt.

Die Aufgabe wird durch die Erfindung gelöst.

Gegenstand der Erfindung ist ein Verfahren zur Herstellung von Organopolysiloxangelen indem
(la) ungesättigte Organopolysiloxanharze aus Einheiten der Formeln

   SiO₂

   (Q-Einheiten) und

   R₃SiO_{1/2}

   und

   R₂R¹SiO_{1/2}

   (M-Einheiten), wobei
   R gleich oder verschieden sein kann und einen einwertigen, gegebenenfalls substituierten Kohlenwasserstoffrest mit 1 bis 18 Kohlenstoffatomen je Rest bedeutet,
   R¹ einen einwertigen Kohlenwasserstoffrest bedeutet, an den Si-H-Gruppen in einer Hydrosilylierungsreaktion angelagert werden können, vorzugsweise einen einwertigen eine terminale, aliphatische C-C-Mehrfachbindung aufweisenden Kohlenwasserstoffrest mit 2 bis 18 Kohlenstoffatomen, bevorzugt ein ω-Alkenylrest mit 2 bis 12 C-Atomen, insbesondere ein Vinylrest ist,
   mit der Maßgabe, dass die Organopolysiloxanharze mindestens 2 Reste R¹, vorzugsweise mindestens 3 Reste R¹, enthalten und dass das molare Verhältnis von M-Einheiten zu Q-Einheiten im Bereich von 0,5 bis 4,0, vorzugsweise im Bereich von 0,5 bis 2,0, liegt, die Organopolysiloxanharze neben den M- und Q-Einheiten noch geringe Mengen an RSiO_{3/2} (T)-Einheiten oder R₂SiO_{2/2} (D)-Einheiten, in Mengen von vorzugsweise 0,01 bis 20 Mol%, bezogen auf die Summe aller Siloxaneinheiten, enthalten können und die Organopolysiloxanharze bis zu 10 Gew.-% freie Si-gebundene Hydroxy- oder Alkoxygruppen enthalten können, und gegebenenfalls
(1b) Verbindungen, die eine polare organische Gruppe, vorzugsweise Glycosidgruppe, wie Oligo- oder Polysaccharidgruppe, Polyoxyalkylengruppe, wie Polyoxyethylen- oder Polyoxypropylengruppe, Hydroxy-, Amid- oder Carboxygruppe und eine hydrosilylierbare Endgruppe aufweisen, mit
(2) einem Gemisch aus zwei Si-H-haltigen Organopolysiloxanen, die unterschiedliche mittlere Kettenlängen aufweisen, der Formel

   (R²₃₋ₓHₓSiO_{1/2})(R²₂SiO_{2/2})ₐ(R²HSiO_{2/2})_{b}(R²₃₋ₓHₓSiO_{1/2}) (I),

   wobei R² gleich oder verschieden ist und einen unsubstituierten oder gegebenenfalls mit Heteroatomen substituierten, aliphatischen, cycloaliphatischen oder aromatischen, ggf. polycyclischen, C₁-C₁₈-Kohlenwasserstoffrest bedeutet,
   × 0 oder 1 ist,
   a und b jeweils ganze Zahlen ≥ 0 sind,
   mit der Maßgabe, dass die Summe a+b ≥ 30 ist,
   dass die Organopolysiloxane durchschnittlich mindestens 2 Si-gebundene H-Atome, vorzugsweise mindestens 3 Si-gebundene H-Atome, enthalten und dass das längerkettige der beiden Organopolysiloxane mindestens die 3-fache Kettenlänge (a+b) des kurzkettigen Organopolysiloxans aufweist,
   in Gegenwart von
(3) die Anlagerung von Si-gebundenem Wasserstoff an
   aliphatische Mehrfachbindungen fördernden Katalysatoren umgesetzt werden,
   wobei (1a), ggf. (1b) und (2) in
(4) Verdünnungsmitteln, vorzugsweise Organopolysiloxanen mit 2 bis 200 Si-Atomen, bevorzugt 2 bis 50 Si-Atomen, oder organischen Verdünnungsmitteln oder Mischungen von Organopolysiloxanen mit 2 bis 200 Si-Atomen, bevorzugt 2 bis 50 Si-Atomen, und organischen Verdünnungsmitteln dispergiert sind
   und die Umsetzungsreaktion durch Zugabe von
(5) als Katalysatorgifte eingesetzten Stopperverbindungen gestoppt wird.

Überraschenderweise wurde gefunden, dass die Verwendung von Si-H-haltigen Organopolysiloxanen unterschiedlicher Kettenlängen eine effektive Maßnahme zur Steuerung der Viskosität von Organopolysiloxangelen eröffnet. Dies war aus US 2012/0202895 A1 nicht zu erwarten.

Vorzugsweise haben die ungesättigten Organopolysiloxanharze (1a) eine Viskosität größer als 0,7 mm²/s bei 25°C. Bevorzugt sind solche Organopolysiloxanharze, die eine Viskosität von größer als 1000 mm²/s bei 25°C haben, oder die Feststoffe sind. Das mit Gelpermeationschromatografie bestimmte gewichtsmittlere Molekulargewicht M_{w} (bezogen auf einen Polystyrolstandard) dieser Organopolysiloxanharze beträgt vorzugsweise 334 bis 200 000 g/mol, bevorzugt 1 000 bis 20 000 g/mol.

Die ungesättigten Organopolysiloxanharze (1a) haben vorzugsweise eine Jodzahl kleiner 254, bevorzugt eine Jodzahl kleiner 76.

Verbindungen (Ib) können eingesetzt werden, um den Organopolysiloxangelen spezielle Eigenschaften, wie etwa Hydrophilie und Wasserquellbarkeit zu verleihen.
Bevorzugt als Verbindungen (Ib) sind solche, die eine Glycosidgruppe, wie Oligo- oder Polysaccharidgruppe, oder eine Polyoxyalkylengruppe, wie Polyoxyethylen- oder Polyoxypropylengruppe, und eine hydrosilylierbare Endgruppe aufweisen. Verbindungen (Ib) werden vorzugsweise in solchen Mengen eingesetzt, dass sie mit einem Gewichtsanteil, bezogen auf das Gesamtgewicht des erhaltenen Organopolysiloxangels von 0,1 bis 10 Gew.-% vorhanden sind.
Solche Gele besitzen eine verbesserte Kompatibilität mit polaren organischen Substanzen und sind sogar in der Lage, äußerst hydrophile Flüssigkeiten, wie Wasser oder Glycerin, aufzunehmen, ohne die viskose Gelstruktur zu verlieren.

Im Rahmen dieser Erfindung soll die Formel (I) für die Si-H-haltigen Organopolysiloxane so verstanden werden, dass die Einheiten der Formeln (R²₂SiO_{2/2})ₐ und (R²HSiO_{2/2})_{b} in beliebiger Weise im Organopolysiloxanmolekül verteilt sein können, dass also sowohl gleiche Einheiten blockweise aufeinanderfolgen können als auch, dass eine mehr oder weniger statistische Abfolge der Einheiten mit beliebigem Wechsel vorliegen kann.

Die Si-H-haltigen Organopolysiloxane gemäß Formel (I) enthalten Si-gebundenen Wasserstoff in Mengen von 0,010 bis 1,60 Gew.-%, vorzugsweise von 0,010 - 1,30 Gew.-%, bevorzugt von 0,010 - 1,25 Gew.-%, insbesondere von 0,010 - 0,60 Gew.-%, wobei die Mengen an Si-gebundenem Wasserstoff für die beiden unterschiedlich langen Si-H-haltigen Organopolysiloxane unterschiedlich sein können aber nicht sein müssen. Wenn die Mengen an Si-gebundenem Wasserstoff für die beiden unterschiedlich langen Si-H-haltigen Organopolysiloxane verschieden sind, gibt es keine Bevorzugung dafür, welches der beiden unterschiedlich langen Si-H-haltigen Organopolysiloxane die größere oder kleinere Menge Si-gebundenen Wasserstoff enthält.

Die Si-gebunden Wasserstoffatome in Formel (I) können entweder nur an terminalen Si-Atomen, nur an kettenständigen Si-Atomen oder sowohl an kettenständigen als auch terminalen Si-Atomen gebunden sein.

Beim Rest R² in Formel (I) sind Heteroatome ausgeschlossen, die die Hydrosilylierungsreaktion behindern würden, wie N- und S-Atome.

Vorzugsweise ist in Formel (I) die Summe a+b ≥ 40, bevorzugt ist die Summe a+b ≥ 55.

Vorzugsweise weist das längerkettige der beiden Organopolysiloxane in (2) mindestens die 3,1-fache Kettenlänge (a+b), bevorzugt die 3,2-fache Kettenlänge, insbesondere die 3,4-fache Kettenlänge, des kurzkettigen Organopolysiloxans auf. Beispiele für Reste R sind Alkylreste, wie der Methyl-, Ethyl-, n-Propyl-, iso-Propyl-, 1-n-Butyl-, 2-n-Butyl-, iso-Butyl-, tert.-Butyl-, n-Pentyl-, iso-Pentyl-, neo-Pentyl-, tert.-Pentylrest, Hexylreste, wie der n-Hexylrest, Heptylreste, wie der n-Heptylrest, Octylreste, wie der n-Octylrest und iso-Octylreste, wie der 2,2,4-Trimethylpentylrest, Nonylreste, wie der n-Nonylrest, Decylreste, wie der n-Decylrest, Dodecylreste, wie der n-Dodecylrest, und Octadecylreste, wie der n-Octadecylrest; Cycloalkylreste, wie Cyclopentyl-, Cyclohexyl-, Cycloheptyl- und Methylcyclohexylreste; Arylreste, wie der Phenyl-, Naphthyl-, Anthryl- und Phenanthrylrest; Alkarylreste, wie o-, m-, p-Tolylreste, Xylylreste und Ethylphenylreste; und Aralkylreste, wie der Benzylrest, der α- und der β-Phenylethylrest.

Beispiele für substituierte Reste R sind Halogenalkylreste, wie der 3,3,3-Trifluor-n-propylrest, der 2,2,2,2',2',2'-Hexafluorisopropylrest, der Heptafluorisopropylrest und Halogenarylreste, wie der o-, m- und p-Chlorphenylrest.

Beispiele für Reste R gelten in vollem Umfang für Reste R².

Bevorzugt handelt es sich beim Rest R sowie beim Rest R² um einen einwertigen Kohlenwasserstoffrest mit 1 bis 6 Kohlenstoffatomen, wobei der Methylrest besonders bevorzugt ist.

Beispiele für Reste R¹ sind Alkenylreste, wie der Vinyl-, 5-Hexenyl-, Cyclohexenyl-, 1-Propenyl-, Allyl-, 3-Butenyl- und 4-Pentenylrest, und Alkinylreste, wie der Ethinyl-, Propargyl- und 1-Propinylrest. Bevorzugt handelt es sich bei dem Rest R¹ um Alkenylreste, besonders bevorzugt um ω-Alkenylreste, insbesondere um den Vinylrest.

Beim Verfahren zur Herstellung der erfindungsgemäßen Organopolysiloxangele werden ungesättigte Organopolysiloxanharze (1a) und ggf. Verbindungen (1b) in Mengen von vorzugsweise 4,5 bis 0,1 Mol, bevorzugt 2 bis 0,8 Mol, besonders bevorzugt 1,8 bis 1,1 Mol, Kohlenwasserstoffrest mit aliphatischer C-C-Mehrfachbindung je Mol Si-gebundenem Wasserstoff in den Si-H-haltigen Organopolysiloxanen (2) eingesetzt.

Die erfindungsgemäßen Gele weisen ein exzellentes Hautgefühl auf. Überraschenderweise wurde gefunden, dass bei Anwendung des erfindungsgemäßen Verfahrens die Steuerung der Viskosität der Organopolysiloxangele durch die Variation des Mischungsverhältnisses der beiden unterschiedlich langen Si-H-haltigen Organopolysiloxane möglich ist. Insbesondere bedeutet dies, dass man bei konstanter Konzentration des Organopolysiloxangelnetzwerkes und bei ansonsten identischen Reaktionsbedingungen bei der Synthese der Organopolysiloxangele, d.h. bei identischer Vorgehensweise, bei identischer Temperatur und Temperaturführung, bei identischen Dosierraten, etc. mit einer größeren Menge des längeren Si-H-haltigen Organopolysiloxans zu einer höheren Viskosität des jeweiligen Gels gelangt. Variiert man die Reaktionsbedingungen, bleibt dieser Effekt erhalten. Wie später in den Beispielen gezeigt wird, bedeutet dies, dass, wenn sich die Viskosität eines Organopolysiloxangels durch die Reduzierung der Aufheizrate reduziert, die Viskosität des Organopolysiloxangels, das einen höheren Anteil an längerkettigem H-Siloxan enthält, immer noch höher als die Viskosität des Organopolysiloxangels mit dem höheren Anteil an kurzkettigerem H-Siloxan wird. Dadurch wird der als erfindungsgemäße Aufgabe zugrundeliegende Effekt erreicht, dass man eine bestimmte Zielviskosität selbst bei einer die Viskosität beeinflussenden Variation der Reaktionsbedingungen einstellen kann, ohne eine Änderung an der Chemie des Organopolysiloxannetzwerkes durchzuführen, die so signifikant ist, dass sie das Organopolysiloxangel und seine Eigenschaften grundlegend ändert. In der Tat wurde gefunden, dass sich die Anwendungseigenschaften von Organopolysiloxangelen bei Variation des Verhältnisses der beiden unterschiedlich langen H-Siloxane so marginal unterscheiden, dass sie als performanceäquivalent verwendbar sind. Dabei ist vor dem Vergleich der Performanceeigenschaften stets darauf zu achten, dass die zu vergleichenden Organopolysiloxangele ggf. durch die Zugabe von Verdünnungsmittel auf die gleiche Viskosität eingestellt werden, da die Viskosität selbst einen erheblichen Einfluss auf das festzustellende Hautgefühl hat. Dieser Effekt muss daher ausgeschlossen werden, bevor der Vergleich der Eigenschaften der beiden Organopolysiloxangele erfolgt.

Zu bemerken ist in diesem Zusammenhang auch, dass der jeweilige Festgehalt des Organopolysiloxangels einen Einfluss auf die Anwendungseigenschaften besitzt. Der Festgehalt ist dabei die Konzentration des Organopolysiloxangelnetzwerkes, das aus der Hydroysilylierungsreaktion erhalten wird im Verdünnungsmittel. Grundsätzlich kann man die Viskosität eines Organopolysiloxangels auch durch die Erhöhung des Festgehaltes steigern, ohne eine Änderung an der Chemie der Hydrosilylierungsreaktion vorzunehmen. Dies geht allerdings wieder zu Lasten der Anwendungseigenschaften. Bei Anwendung des erfindungsgemäßen Verfahrens bei einer die Viskosität beeinflussenden Variation der Reaktionsbedingungen, beispielsweise die Aufheizrate, wird die Zielviskosität durch die Anpassung des Verhältnisses der beiden unterschiedlichen H-Siloxane erreicht, wobei der Festgehalt des Organopolysiloxangels konstant gehalten wird.

Eine wesentliche Voraussetzung dafür, dass dies alles möglich ist, besteht darin, dass der Kettenlängenunterschied zwischen den beiden H-Siloxanen ausreichend groß ist.

Es wurde gefunden, dass die Kettenlänge des längerkettigen H-Siloxans mindestens das 3-fache der Kettenlänge des kürzerkettigen H-Siloxans betragen muss, wobei die Kettenlänge des kürzerkettigen H-Siloxans bevorzugt mindestens 40, insbesondere mindestens 55 Siloxaneinheiten a+b in Formel (I) beträgt.

Im Rahmen der experimentellen Arbeiten erwiesen sich beispielsweise die Kombination zweier H-Siloxane mit den Kettenlängen 75 und 225 sowie 140 und 450 als besonders wirkungsvoll.

Besonders wirksam waren dabei solche H-Siloxane der Formel (I), die nur kettenständige Si-H-Einheiten b enthielten und x gleich 0 ist, also solche der Formel (I'):

(R²₃SiO_{1/2})(R²₂SiO_{2/2})ₐ(R²HSiO_{2/2})_{b}(R²₃SiO_{1/2}) (I'),

wobei R², a und b die oben dafür angegebene Bedeutung haben.

Beispiele für bevorzugte kurzkettige H-Siloxane sind solche für die die Reste R² in Formel (I') Methylreste sind und in denen das Verhältnis a:b die in der nachfolgenden Tabelle 1 angegebenen Werte besitzt:

**Tabelle 1: Kurzkettige Si-H-haltige Polydimethylsiloxane**

| Laufende Nummer | Verteilung a:b | Kettenlänge (a+b) |
|---|---|---|
| 1 | a = 0 | 55 |
| 2 | 0,3 : 1 | 45 |
| 3 | 0,5:1 | 60 |
| 4 | 1:1 | 140 |
| 5 | 2:1 | 140 |
| 6 | 3,5:1 | 45 |
| 7 | 5:1 | 75 |
| 8 | 6:1 | 65 |
| 9 | 7:1 | 80 |
| 10 | 9:1 | 60 |
| 11 | 15:1 | 75 |
| 12 | 20:1 | 75 |
| 13 | 25:1 | 85 |
| 14 | 50:1 | 140 |

Beispiele für bevorzugte langkettige H-Siloxanäquilibrate sind solche, für die die Reste R² in Formel (I') Methylreste sind, und in denen das Verhältnis a:b die in der nachfolgenden Tabelle 2 angegebenen Werte besitzt:

**Tabelle 2: Langkettige Si-H-haltige Polydimethylsiloxane**

| Laufende Nummer | Verteilung a:b | Kettenlänge (a+b) |
|---|---|---|
| 15 | a = 0 | 255 |
| 16 | 0,3 : 1 | 225 |
| 17 | 0,5:1 | 360 |
| 18 | 1:1 | 440 |
| 19 | 2:1 | 275 |
| 20 | 3,5:1 | 365 |
| 21 | 5:1 | 445 |
| 22 | 6:1 | 360 |
| 23 | 7:1 | 375 |
| 24 | 9:1 | 465 |
| 25 | 15:1 | 225 |
| 26 | 20:1 | 465 |
| 27 | 25:1 | 365 |
| 28 | 50:1 | 450 |

Als Katalysator (3) können bei dem erfindungsgemäßen Verfahren die gleichen Katalysatoren eingesetzt werden, die auch bisher zur Förderung der Anlagerung von Si-gebundenem Wasserstoff an aliphatische Mehrfachbindungen eingesetzt werden konnten. Bei den Katalysatoren handelt es sich vorzugsweise um ein Metall aus der Gruppe der Platinmetalle oder um eine Verbindung oder einen Komplex aus der Gruppe der Platinmetalle. Beispiele für solche Katalysatoren sind metallisches und feinverteiltes Platin, das sich auf Trägern wie Siliciumdioxid, Aluminiumoxid oder Aktivkohle befinden kann, Verbindungen oder Komplexe von Platin, wie Platinhalogenide, z. B. PtCl₄, H₂PtCl₆•6H₂O, Na₂PtCl₄•₄H₂0, Platin-Olefin-Komplexe, Platin-Alkohol-Komplexe, Platin-Alkoholat-Komplexe, Platin-Ether-Komplexe, Platin-Aldehyd-Komplexe, Platin-Keton-Komplexe, einschließlich Umsetzungsprodukten aus H₂PtCl₆•6H₂0 und Cyclohexanon, Platin-Vinyl-siloxankomplexe, wie Platin-1,3-Divinyl-1,1,3,3-tetramethyl-disiloxankomplexe mit oder ohne Gehalt an nachweisbarem anorganisch gebundenen Halogen, Bis-(γ-picolin)-platindichlorid, Trimethylendipyridinplatindichlorid, Dicyclopentadienplatindichlorid, Dimethylsulfoxidethylenplatin-(II)-dichlorid, Cyclooctadien-Platindichlorid, Norbornadien-Platindichlorid, γ-picolin-Platindichlorid, Cyclopentadien-Platindichlorid, sowie Umsetzungsprodukte von Platintetrachlorid mit Olefin und primärem Amin oder sekundärem Amin oder primärem und sekundärem Amin, wie das Umsetzungsprodukt aus in 1-0cten gelöstem Platintetrachlorid mit sec.-Butylamin oder Ammonium-Platinkomplexe. Bevorzugte Hydrosilylierungskatalysatoren sind Platinverbindungen, die in einem zur Verwendung in kosmetischen Formulierungen geeigneten Lösungsmittel vorliegen.

Bevorzugt wird der Katalysator (3) in Mengen 1 bis 100 Gew.-ppm (Gewichtsteilen je Million Gewichtsteilen) eingesetzt, berechnet als elementares Platin und bezogen auf das Gesamtgewicht der Komponenten (1a), ggf. (1b) und (2).

Es hat sich gezeigt, dass sich eine zu große Menge Platinkatalysator nachteilig auf die sensorischen Eigenschaften der erhaltenen Organopolysiloxangele auswirkt. Insbesondere wird die Eigenschaft Krümeligkeit nachteilig beeinflusst. Außerdem wird durch eine zu große Menge Platinkatalysator eine nachteilige Gelbfärbung der Gele begünstigt, abgesehen davon, dass die Verwendung einer größeren Menge Platin als der unbedingt benötigten aufgrund des hohen Preises, der für solche Katalysatoren zu zahlen ist, wirtschaftliche Nachteile nach sich zieht. Daher wird eine Menge von mehr als 100 Gew.-ppm elementares Platin, bezogen auf das Gesamtgewicht der Komponenten (1a), ggf. (1b) und (2), bevorzugt ausgeschlossen, obwohl das Verfahren auch mit größeren Platinmengen grundsätzlich zu Organopolysiloxangelen führt. Da diese jedoch keinen oder einen nur sehr eingeschränkten Nutzen für das Zielanwendungsfeld Kosmetik besitzen, ist deren Einbezug in den erfindungsgemäßen Rahmen nicht bevorzugt.

Es hat sich insbesondere gezeigt, dass für Hydrosilylierungsreaktionen, die unter Anwendung von Hitze ablaufen, größere Mengen Platinkatalysator benötigt werden als für solche Hydrosilylierungen, die ohne die Anwendung von extern zugeführter Hitze bereits bei Raumtemperatur, also bei 20 - 30°C, ablaufen. Für Hydrosilylierungen unter Anwendungen von Hitze haben sich Platinmengen von 10 - 90 Gew.-ppm, besonders von 15 - 80 Gew.-ppm und insbesondere von 20 - 75 Gew.-ppm als vorteilhaft erwiesen. Für Reaktionen, die bereits in der "Kälte", also bei Raumtemperatur, ablaufen und sich lediglich durch die Entwicklung exothermer Reaktionswärme selbst aufheizen, haben sich Platinmengen von 1 - 60 Gew.-ppm, besonders von 2 - 55 Gew.-ppm, insbesondere von 3 - 50 Gew.-ppm als vorteilhaft und ausreichend erwiesen.

Die erfindungsgemäßen Organopolysiloxangele enthalten vorzugsweise 1 bis 98 Gew.-% Verdünnungsmittel, bevorzugt 50 bis 95 Gew.-% Verdünnungsmittel, bezogen auf das Gesamtgewicht der Organopolysiloxangele.

Nicht-reaktive oder relativ nicht-reaktive Verdünnungsmittel sind bevorzugt. Im Rahmen der vorliegenden Erfindung wird der Begriff "nicht-reaktiv" in Bezug auf die in Frage stehende Vernetzungsreaktion und die hierin eingesetzten Reaktanden verwendet. Ein relativ nicht-reaktives Verdünnungsmittel ist weniger als ein Zehntel so reaktiv mit den Reaktanden der Vernetzungsreaktion im Vergleich zu den Reaktanden untereinander in der Vernetzungsreaktion.

Geeignete Beispiele von Verdünnungsmittel beinhalten cyclische und lineare Organopolysiloxane, organische Verdünnungsmittel oder Mischungen von Organopolysiloxanen und organischen Verdünnungsmitteln.

Das als Verdünnungsmittel eingesetzte Organopolysiloxan kann ein einzelnes Organopolysiloxan oder eine Mischung von Organopolysiloxanen sein. Das Organopolysiloxan kann Alkyl-, Aryl-, Alkaryl- und Aralkylgruppen tragen. Solche Organopolysiloxane können beispielhaft durch Polydimethylsiloxan, Polydiethylsiloxan, Polymethylethylsiloxan, Polymethylphenylsiloxan und Polydiphenylsiloxan angegeben werden, sind aber nicht darauf beschränkt.

Möglich ist auch die Verwendung von funktionellen Organopolysiloxanen als Verdünnungsmittel, beispielsweise acrylamidfunktionelle Siloxanfluide, acrylfunktionelle Siloxanfluide, amidfunktionelle Siloxanfluide, aminofunktionelle Siloxanfluide, carbinolfunktionelle Siloxanfluide, carboxyfunktionelle Siloxanfluide, chloralkylfunktionelle Siloxanfluide, epoxfunktionelle Siloxanfluide, glykolfunktionelle Siloxanfluide, ketalfunktionelle Siloxanfluide, mercaptofunktionelle Siloxanfluide, methylesterfunktionelle Siloxanfluide, perfluorofunktionelle Siloxanfluide und silanolfunktionelle Siloxane.

Cyclische Polydimethylsiloxane als Verdünnungsmittel können beispielhaft Hexamethylcyclotrisiloxan, Octamethylcyclotetrasiloxan, Decamethylcyclopentasiloxan und Dodecamethylcyclohexasiloxan sein, sind aber nicht darauf beschränkt.

Vorzugsweise ist das als Verdünnungsmittel eingesetzte Organopolysiloxan ein Polydimethylsiloxan mit 2 bis 200 Si-Atomen, bevorzugt 2 bis 50 Si-Atomen, besonders bevorzugt sind lineare Polydimethylsiloxane mit einer Viskosität von 1,5 bis 50 mm²/s bei 25°C.

Als organische Verdünnungsmittel können aromatische Kohlenwasserstoffe, Alkohole, Aldehyde, Ketone, Amine, Ester, Ether, Alkylhalogenide oder aromatische Halogenide verwendet werden. Stellvertretende Beispiele sind Alkohole, wie Methanol, Ethanol, i-Propanol, Cyclohexanol, Benzylalkohol, 2-Octanol, Ethylenglykol, Propylenglykol und Glycerin; aliphatische Kohlenwasserstoffe, wie Pentan, Cyclohexan, Heptan, Lackbenzine; Alkylhalogenide wie Chloroform, Kohlenstofftetrachlorid, Perchlorethylen, Ethylchlorid und Chlorbenzol; aromatische Kohlenwasserstoffe, wie Benzol, Toluol, Ethylbenzol und Xylol; Ester von Carbonsäuren mit 2 bis 30 C-Atomen, wie Ethylacetat, Isopropylacetat, Ethylacetoacetat, Amylacetat, Isobutylisobutyrat, Benzylacetat, Isopropylpalmitat und Isopropylmyristat (=Myristinsäure-isopropylester); Ether, wie Ethylether, n-Butylether, Tetrahydrofuran und 1,4-Dioxan; Ketone, wie Aceton, Methylethylketon, Cyclohexanon, Diacetonalkohol, Methylamylketon und Diisobutylketon; Fettöle einschließlich mehrfach ungesättigter ω-3- und ω-6-Fettsäuren, und deren Ester; pflanzliche Öle, wie Erdnuss-, Oliven-, Palm-, Canola-, Maiskeim-, Soja-, Sonnenblumenöl und dergleichen; und natürliche und synthetische Öle oder öllösliche Feststoffe, wie verschiedene Mono-, Di- und Triglyceride, polyoxyalkylierte pflanzliche Öle, Lanolin, Lecithin und dergleichen; und Erdölkohlenwasserstoffe, wie Petrolatum, Mineralöl, Benzin, Petrolether. Diese Beispiele dienen der Erläuterung und sind nicht als Einschränkung zu verstehen.

Andere gemischte organische Verdünnungsmittel können ebenfalls verwendet werden, wie Acetonitril, Nitromethan, Dimethylformamid, Propylenoxid, Trioctylphosphat, Butyrolacton, Furfural, Pinienöl, Terpentin und m-Cresol.

Geeignete organische Verdünnungsmittel sind auch flüchtige Aromastoffe, wie Pfefferminzöl, Öl von grüner Minze, Menthol, Vanille, Zimtöl, Nelkenöl, Lorbeeröl, Anisöl, Eukalyptusöl, Thymianöl, Zedernöl, Öl von der Muskatnuss, Öl von Salbei, Kassiaöl, Kakao, Süßholzsaft, Stärkezuckersirup aus Mais mit hohem Fructosegehalt, Citrusöle, wie Zitrone, Orange, Limone und Grapefruit, Fruchtessenzen, wie Apfel, Birne, Pfirsich, Traube, Erdbeere, Himbeere, Kirsche, Pflaume, Ananas und Aprikose; und andere nützliche Aromastoffe einschließlich Aldehyde und Ester, wie Zimtsäureessigester, Zimtaldehyd, Eugenylformat, p-Methylanisol, Acetaldehyd, Benzaldehyd, Anisaldehyd, Citral, Neral, Decanal, Vanillin, Tolylaldehyd, 2,6-Dimethyloctanal und 2-Ethylbutyraldehyd.

Ein Teil oder das gesamte organische Verdünnungsmittel kann ein oder mehrere flüchtige Duftstoffe umfassen, wie natürliche Produkte und Parfumöle. Einige stellvertretende natürliche Produkte und Parfumöle sind Amber, Benzoin, Zibet, Nelke, Zedernöl, Jasmin, Mate, Mimose, Moschus, Myrrhe, Iris, Sandelholzöl und Vetiveröl; Aromachemikalien, wie Amylsalicylat, Amylzimtaldehyd, Benzylacetat, Citronellol, Cumarin, Geraniol, Isobornylacetat, Ambrette und Terpinylacetat, und verschiedene klassische Parfümölfamilien, wie die Blumenbouquetfamilie, die orientalische Familie, die Chyprefamilie, die Holzfamilie, die Citrusfamilie, die Canoefamilie, die Lederfamilie, die Gewürzfamilie und die Kräuterfamilie.

Das organische Verdünnungsmittel kann auch aliphatische oder alicyclische Kohlenwasserstoffe mit 4 bis 30 C-Atomen, vorzugsweise gesättigte Kohlenwasserstoffe, umfassen. Die aliphatischen Kohlenwasserstoffe können geradkettig oder verzweigt sein, und die alicyclischen Kohlenwasserstoffe können unsubstituierte cyclische Kohlenwasserstoffe oder aliphatische kohlenwasserstoffsubstituierte Kohlenwasserstoffe darstellen. Beispiele für geeignete Kohlenwasserstoffe sind n-Heptan, n-Octan, Isooctan, n-Decan, Isodecan, n-Dodecan, Isododecan, Cyclohexan, Cycloheptan, Cyclooctan, Methylcyclohexan, Dimethylcyclohexan, Ethylcyclohexan, Nonylcyclohexan und dergleichen. Auch diese Aufzahlung dient der Erläuterung und ist nicht als Einschränkung zu verstehen.

Weitere geeignete organische Verdünnungsmittel sind ölartige Polyether, wie Bis(alkyl)ether von niedermolekularen Glykolen, und flüssige oligomere und polymere Polyoxyalkylenglykole, deren Alkylmono- und -diether und Mono- und Dialkylester, wobei deren Verwendung aber nicht bevorzugt ist. Vorzugsweise wird der überwiegende Teil der Polyoxyalkylenglykole aus einem überwiegenden Teil (> 50 Mol-%) von Alkylenoxiden mit mehr als zwei Kohlenstoffatomen, d. h. Propylenoxid, 1,2- und 2,3-Butylenoxid, Tetrahydrofuran, Oxetan, Cyclohexenoxid und dergleichen, hergestellt.

Bevorzugte organische Verdünnungsmittel weisen eine Viskosität im Bereich von 0,5 bis 200 mm²/s (25°C) auf, wobei solche Verdünnungsmittel mit einem Siedepunkt im Bereich von 50°C bis 300°C besonders bevorzugt sind.

Es können zahlreiche Mischungen von Verdünnungsmitteln verwenden werden, die lediglich auf diejenigen Zusammensetzungen beschränkt sind, bei denen nach der Herstellung des erfindungsgemäßen Organopolysiloxangels keine Phasentrennung auftritt.

Die Herstellung des Gels ist leicht durchführbar. Im Allgemeinen gibt man in einer Art Eintopfverfahren alle Bestandteile außer dem Katalysator zu, rührt langsam, bis sich das ggf. pulverförmige ungesättigte Organopolysiloxanharz gelöst hat und setzt dann unter kontinuierlichem Rühren den Katalysator zu. Die Zusammensetzung kann bei Raumtemperatur belassen werden, bis sich ein Gel gebildet hat, oder sie kann erhitzt werden.
Vorzugsweise wird die Zusammensetzung auf eine Temperatur zwischen 50°C und 130°C, bevorzugt zwischen 70°C und 120°C erhitzt, bis die Mischung geliert oder fest wird. Die Gelierung erfolgt vorzugsweise innerhalb von zehn Stunden, bevorzugt innerhalb von drei Stunden.

Die Reaktion wird am Ende der Reaktionszeit durch Zugabe vorzugsweise eines Hydrosilylierungskatalysatorgiftes als Stopperverbindung (5) abgebrochen, wodurch die Nachhärtung, die durch verbleibende vernetzende Hydrosilylierungsreaktionen, die in den Siliconelastomeren auftreten, bewirkt wird, beendet wird. Als Stopper werden dabei Katalysatorgifte eingesetzt, die den Hydrosilylierungskatalysator irreversibel verändern und dadurch deaktivieren.
Die Stopperverbindungen (5) werden vorzugsweise in Mengen von mindestens 1,1 Mol die Stopperung bewirkende funktionelle Gruppe, vorzugsweise Mercaptogruppe, je Mol elementares Platin, im Katalysator (3) eingesetzt.
Es hat sich dabei als besonders vorteilhaft erwiesen, eine deutlich überstöchiometrische Menge des Stoppers einzusetzen, da dies eine positive Wirkung auf den Langzeiterhalt der Cremigkeit des fertigen Gels hat.

Beispielhaft für Stopperverbindungen (5), welche geeignet sind, um die Nachhärtung zu beenden, sind schwefelorganische Verbindungen, wie Mercaptogruppen aufweisende organische Verbindungen. Weitere geeignete Verbindungen sind in US 6,200,581 genannt. Bevorzugte Hydrosilylierungskatalysatorgifte als Stopperverbindungen (5) sind Mercaptoalkylgruppen aufweisende Organopolysiloxane, besonders bevorzugt sind 3-Mercaptopropylgruppen aufweisende Organopolysiloxane, wie mercaptopropylfunktionelle Silsesquisiloxane oder mercaptopropylfunktionelle Polyorganosiloxane.

Als Stopperverbindungen (5) eingesetzte Mercaptoalkylgruppen aufweisende Organopolysiloxane werden vorzugsweise in Mengen von 200 bis 1,1 Mol, bevorzugt 50 bis 1,5 Mol, besonders bevorzugt 20 bis 2,0 Mol, Mercaptogruppen je Mol elementares Platin im Katalysator (3) eingesetzt.

Bei dem erfindungsgemäßen Verfahren werden Organopolysiloxangele, welche für die Anwendung in kosmetischen Formulierungen geeignet sind, erhalten.

Es ist auch möglich, das Verfahren in zwei Teilschritten auszuüben, wobei im Wesentlichen im ersten Teilschritt die Hydrosilylierung mit dem ersten der beiden unterschiedlich langen Si-H-haltigen Organopolysiloxane stattfindet und im zweiten Teilschritt die Hydrosilylierung mit dem zweiten Si-H-haltigen Organopolysiloxan. Im Prinzip spielt es keine Rolle, ob zuerst das kurzkettige oder das langkettige Si-H-haltige Organopolysiloxan zur Hydrosilylierung eingesetzt wird, allerdings ist es bevorzugt, dass im ersten Teilschritt mit dem kurzkettigen und im zweiten Teilschritt mit dem langkettigen Si-H-haltigen Organopolysiloxan hydrosilyliert wird. Da mit dem kurzkettigen Si-H-haltigen Organopolysiloxan ein niederviskoseres Vorprodukt entsteht, ist es bei der bevorzugten Vorgehensweise leichter, das zweite Si-H-haltige Organopolysiloxan im zweiten Teilschritt homogen zu dispergieren.

Die Vorgehensweise beim Verfahren in Teilschritten unterscheidet sich von der Vorgehensweise im Eintopfverfahren insofern, dass nicht die gesamte Masse aller Edukte von Anfang an eingesetzt wird. Man mischt zunächst das zuerst verwendete Si-H-haltige Organopolysiloxan, vorzugsweise das kurzkettige, mit der gesamten Menge des ungesättigten
Organopolysiloxanharzes (1a) und dem Verdünnungsmittel (4). Falls ein pulverförmiges, ungesättigtes Organopolysiloxanharz eingesetzt wird, wird es entweder vorab in einem Verdünnungsmittel gelöst, oder man setzt es der Reaktionsmischung pulverförmig zu und dispergiert diese dann solange, bis sich das Harz vollständig gelöst hat. Anschließend wird unter kontinuierlichem Rühren der Katalysator zugesetzt. Hierbei kann sowohl die gesamte für die Reaktion erforderliche Katalysatormenge zugesetzt werden als auch nur eine Teilmenge desselben. Vorzugsweise wird erhitzt auf eine Temperatur zwischen 50°C und 130°C, bevorzugt zwischen 70°C und 120°C, bis die Mischung geliert oder fest wird. Die Gelierung erfolgt vorzugsweise innerhalb von zehn Stunden, bevorzugt innerhalb von drei Stunden.
Danach wird im zweiten Teilschritt der Gelherstellung das zweite Si-H-haltige Organopolysiloxan, vorzugsweise das langkettige, unter Rühren zugegeben. Falls noch nicht die gesamte Katalysatormenge zugegeben wurde, wird der Rest der benötigten Katalysatormenge nun zugegeben und weiter gerührt, bis die Mischung geliert oder fest wird. Die Gelierung erfolgt vorzugsweise innerhalb von zehn Stunden, bevorzugt innerhalb von drei Stunden. Die Reaktion wird am Ende der Reaktionszeit durch Zugabe eines Stoppers abgebrochen, wie bereits weiter oben ausgeführt. Es werden Organopolysiloxangele, welche für die Anwendung in kosmetischen Formulierungen geeignet sind, erhalten.

Optional kann je nach gewähltem Verfahren in einem zweiten oder dritten optionalen Verfahrensschritt eine Verdünnung des zunächst erhaltenen Organopolysiloxanbasisgels erfolgen.

Dabei kommen hochscherende Mischtechniken nach Stand der Technik zum Einsatz.

Das Verdünnen kann durch intensives Mischen und Dispergieren mit geeigneten Rühraggregaten oder in Rotor-Stator-Rührvorrichtungen, Kolloidmühlen, Hochdruckhomogenisatoren, Mikrokanälen, Membranen, Strahldüsen und ähnlichem, oder mittels Ultraschall erfolgen. Dabei werden Homogenisiergeräte und Verfahren gemäß Stand der Technik eingesetzt. Homogenisiergeräte und Verfahren sind z.B. in Ullmann's Encyclopedia of Industrial Chemistry, CD-ROM-Ausgabe 2003, Wiley-VCH Verlag, unter dem Stichwort "Emulsions" beschrieben.

In dem optionalen Verdünnungsschritt des Organopolysiloxanbasisgels ist es möglich, eine Vielzahl unterschiedlicher Gele herzustellen, die in ihrer Konsistenz und ihrem Eigenschaftsprofil in einem breiten Bereich variieren. Man kann dabei das gleiche Verdünnungsmittel verwenden, welches im ersten bzw. den ersten beiden Verfahrensschritten verwendet worden ist, oder ein zweites Verdünnungsmittel, das hiervon verschieden ist. Alternativ kann auch eine beliebige Mischung der vorhergehend hierin beschriebenen Verdünnungsmittel und/oder ein aktiver Wirkstoff für die Körperpflege oder Gesundheitspflege oder eine Mischung eines aktiven Wirkstoffs für die Körperpflege oder Gesundheitspflege mit einem oder mehreren der hierin beschriebenen Verdünnungsmitteln zugegeben werden, mit der Maßgabe, dass keine Phasentrennung auftritt.

Ein "aktiver Wirkstoff für die Körperpflege oder Gesundheitspflege" bedeutet im vorliegenden Zusammenhang eine beliebige Verbindung oder Mischung von Verbindungen, die im Fachgebiet als Zusatzstoffe in Körperpflegeformulierungen bekannt sind und die typischerweise zugesetzt werden, um das Haar oder die Haut zu behandeln, um einen kosmetischen und/oder ästhetischen Nutzen zu erzielen; eine beliebige Verbindung oder Mischung von Verbindungen, die im Fachgebiet bekannt sind, um einen pharmazeutischen oder medizinischen Nutzen zu erzielen; eine beliebige Verbindung, mit der eine pharmakologische Wirksamkeit oder ein sonstiger Effekt bei der Diagnose, Heilung, Linderung, Behandlung oder Vorbeugung von Krankheiten erzielt werden soll, oder um die Struktur oder eine beliebige Funktion des Körpers eines Menschen oder eines Tiers zu beeinflussen; und jede beliebige Verbindung, die bei der Herstellung von Arzneimittelprodukten eine chemische Veränderung erfahren kann und die in Arzneimitteln in modifizierter Form vorliegen kann, um die angegebene Wirksamkeit oder den angegebenen Effekt hervorzurufen.

Die aktiven Wirkstoffe für die Körperpflege oder Gesundheitspflege sind vorzugsweise ausgewählt aus der Gruppe der fett- oder öllöslichen Vitamine, öllöslichen Arzneimittel, Antiaknemittel, antibakterielle Mittel, fungizide Mittel, entzündungshemmende Mittel, schuppenflechtebekämpfende Mittel, Betäubungsmittel, juckreizlindernde Mittel, hautentzündungshemmende Mittel und Mittel, die im Allgemeinen als Sperrfilme betrachtet werden, und öllöslichen UV-Absorber.

Beispiele für nützliche aktive Bestandteile zur Verwendung im ggf. dritten Verfahrensschritt gemäß der Erfindung sind folgende:
Beispiele für öllösliche Vitamine sind, aber nicht beschränkt darauf, Vitamin A₁, RETINOL, C₂- bis C₁₈-Ester von RETINOL, Vitamin E, TOCOPHEROL, Ester von Vitamin E und Mischungen derselben. RETINOL umfasst trans-RETINOL, 13-cis-RETINOL, 11-cis-RETINOL, 9-cis-RETINOL und 3,4-Didehydro-RETINOl. Das öllösliche Vitamin kann in der Zusammensetzung gemäß der Erfindung in Mengen von 0,01 bis 50 Gewichtsprozent verwendet werden.

Es sollte bemerkt werden, dass RETINOL ein International Nomenclature Cosmetic Ingredient Name (INCI), vergeben von The Cosmetic, Toiletry and Fragrance Association (CTFA), Washington DC, für Vitamin A ist. Andere geeignete Vitamine und die INCI-Namen für die in Betracht stehenden Vitamine, die hierin umfasst sind, sind RETINYL ACETAT, RETINYL PALMITATE, RETINYL PROPIONATE, α- TOCOPHEROL,TOCOPHERSOLAN,TOCOPHERYL ACETATE, TOCOPHERYLLINOLEATE, TOCOPHERYLNICOTINATE und TOCOPHERYL SUCCINATE.

Einige Beispiele für kommerziell erhältliche Produkte, die zur Verwendung hierin geeignet sind, sind Vitamin-A-Acetat, Fluka Chemie AG, Buchs, Schweiz; CIOVI-OX T-50, ein Vitamin E-Produkt von Henkel Corporation, La Grange, Illinois; COVI-OX T-70, ein anderes Vitamin-E-Produkt von Henkel Corporation, La Grange, Illinois, und Vitamin-E-Acetat, ein Produkt von Roche Vitamins & Fine Chemicals, Nutley, New Jersey.

Stellvertretende Beispiele für einige geeignete öllösliche Arzneimittel, welche als aktive Bestandteile zugefügt werden können, sind Clonidin, Scopolamin, Propranolol, Estradiol, Phenylpropanolaminhydrochlorid, Ouabain, Atropin, Haloperidol, Isosorbid, Nitroglycerin, Ibuprofen, Ubichinone, Indomethacin, Prostaglandine, Naproxen, Salbutamol, Guanabenz, Labetalol, Pheniramin, Metrifonat und Steroide.

Ebenso hierin umfasst als ein Arzneimittel für die Zwecke der vorliegenden Erfindung sind Antiaknemittel, wie Benzoylperoxid, Triclosan und Tretinoin; antibakterielle Mittel wie Chlorhexidingluconat; fungizide Mittel, wie Miconazolnitrat; entzündungshemmende Mittel, wie Salicylsäure; corticosteroide Arzneimittel; nichtsteroide entzündungshemmende Mittel, wie Diclofenac; schuppenflechtebekämpfende Mittel, wie Clobetasolpropionat und Retinoide, Betäubungsmittel, wie Lidocain; juckreizlindernde Mittel, wie Polidocanol; hautentzündungshemmende Mittel, wie Prednisolon und Mittel, die im Allgemeinen als Sperrfilme betrachtet werden.

Stellvertretende Beispiele für öllösliche UV-Absorber, welche als aktive Bestandteile zugefügt werden können, sind 1-(4-Methoxyphenyl)-3-(4-tert-butylphenyl)propan-1,3-dione (INCI: Butyl Methoxydibenzoylmethan), 2-Ethylhexyl-(2E)-3-(4-methoxyphenyl)prop-2-enoat (INCI: Octyl Methoxycinnamat), 4-Hydroxy-2-methoxy-5-(oxo-phenylmethyl)benzenesulfonic acid (INCI: Benzophenon-4), 2-Hydroxy-4-methoxybenzophenon-5-sulfonsäure Natriumsalz (INCI: Benzophenon-5) und 2-Ethylhexyl-2-hydroxybenzoat(INCI: Ethylhexylsalicylat).

Bevorzugt wird je nach gewähltem Verfahren in einem dritten oder vierten Verfahrensschritt das nach dem ersten oder zweiten oder optionalen dritten Verfahrensschritt erhaltene erfindungsgemäße Organopolysiloxangel unter Verwendung von standardmäßigen hochscherenden Mischtechniken bis zu einer cremigen Konsistenz homogenisiert. Hierfür geeignete Technologien sind die gleichen wie oben für den Verdünnungsschritt genannt. Wenn im optionalen dritten Verfahrensschritt eine zusätzliche Menge Verdünnungsmittel zugegeben worden ist, so wird dieses im vierten Verfahrensschritt homogen im Gel verteilt. Das Gel quillt und verändert seine Weichheit.

Unter cremig in Bezug auf das Gel ist zu verstehen, dass das Ausgangsgel bis zu einer cremigen Konsistenz geschert worden ist. Das resultierende cremige Gel kann je nachdem gießbar oder verhältnismäßig steif sein. Durch das Attribut cremig unterscheiden sich diese gescherten Gele, die transparent oder opak sein können, von den unmittelbar durch Gelierung der reaktiven Bestandteile hergestellten Gelen.

Unter lagerstabil im Rahmen dieser Erfindung ist zu verstehen, dass sich die gebildeten Organopolysiloxangele innerhalb von 6 Monaten Lagerung bei Raumtemperatur nicht in zwei oder mehr Phasen entmischen. Bevorzugt ändert sich die Weichheit des Gels in diesem Zeitraum nicht.

Dem Fachmann ist verständlich, dass das Aufnahmevermögen für Verdünnungsmittel aufgrund der dreidimensionalen Netzwerkstruktur von Organopolysiloxangelen im Allgemeinen beschränkt ist und in Abhängigkeit von der Netzwerkstruktur und Netzwerkzusammensetzung variieren kann. Ist das Aufnahmevermögen für Verdünnungsmittel überschritten, so ist die Bildung einer Verdünnungsmittelphase neben einer Gelphase zu erkennen.

Die erfindungsgemäßen Organopolysiloxangele sind besonders bevorzugt geeignet für kosmetische Anwendungen, und werden daher bevorzugt in kosmetischen Zusammensetzungen eingesetzt. Sie eignen sich aber auch für andere Anwendungen, beispielsweise für medizinische und technische Anwendungen.

Die Organopolysiloxangele haben besonderen Wert in Körperpflegeprodukten. Sie können sanft auf der Haut verteilt werden und können daher alleine verwendet werden oder mit anderen Körperpflegeproduktbestandteilen gemischt werden, um eine Vielzahl von Körperpflegeprodukten zu bilden.

Beispiele von Körperpflegeproduktbestandteilen sind Ester, Wachse, Öle und Fette von tierischem oder pflanzlichem Ursprung, Fettalkohole, Fettsäuren, Alkylester von Fettsäuren, Kohlenwasserstoffe und -wachse, Wasser, organische Lösungsmittel, Parfüme, oberflächenaktive Mittel, öllösliche Vitamine, wasserlösliche Vitamine, öllösliche Arzneimittel, wasserlösliche Arzneimittel, UV-Absorber und aktive pharmazeutische Verbindungen.

Insbesondere sind die erfindungsgemäßen Organopolysiloxangele in Antiperspirantien und Deoperspirantien geeignet, da sie ein trockenes Gefühl zurücklassen und die Haut nicht während der Verdampfung abkühlen. Sie sind gleitfähig und verbessern die Eigenschaften von Hautcremes, Hautpflegelotionen, Moisturizern, Gesichtsbehandlungen, wie z.B. Akne- oder Faltenentfernern, Körper- und Gesichtsreinigern, Badeölen, Parfüms, Kölnisch Wasser, Sachets, Sonnenschutzmittel, Preshave- und Aftershave-Lotionen, flüssigen Seifen, Rasierseifen und Rasierschäumen. Sie können in Haarshampoos, Haarkonditionierern, Haarsprays, Mousses, Dauerwellenmittel, Haarentfernungsmittel und Nagelhautabdeckungen verwendet werden, um Glanz und Trockengleiten zu verbessern und Konditioniervorteile bereitzustellen.

In Kosmetika fungieren Sie als Verteilungsmittel für Pigmente in Make-ups, Farbkosmetika, Grundierungen, Rouge, Lippenstiften, Lippenbalsam, Lidstrich, Mascara, Ölentfernern und Farbkosmetikentfernern. Sie sind als Verabreichungssysteme für hierin beispielhaft genannte öllösliche aktive Bestandteile, wie z.B. Vitamine, Arzneimittel und UV Absorber geeignet. Wenn sie in Stiften, Gelen, Lotionen, Cremes, Roll-ons eingesetzt werden, verleihen die Elastomere ein trockenes, seidig sanftes Gefühl. In Kosmetika und anderen Hautpflegeprodukten eingebracht, verleihen die Elastomere einen Mattierungseffekt.

Zusätzlich zeigen die Organopolysiloxangele eine Vielzahl vorteilhafter Eigenschaften, wie z.B. Klarheit, Lagerstabilität und Einfachheit der Herstellung. Daher haben sie einen breiten Anwendungsbereich, insbesondere in Antiperspirantien, Deodorantien, Hautpflegeprodukten, in Parfüms als Träger und für die Haarkonditionierung, beispielsweise in Hair-Balm oder Hair-Mask Conditioner.

Die Organopolysiloxangele haben Verwendung über den Körperpflegebereich hinaus, einschließlich deren Verwendung als Füllstoff oder Isolierungsmaterial für elektrische Kabel, Boden- oder Wasserbarrieren für Bodenstabilisierung oder als Ersatz für Epoxymaterialen die in Bauteilen in der elektronischen Industrie verwendet werden. Sie sind ebenfalls als Träger für vernetzte Siliconkautschukteilchen geeignet. In diesen Anwendungen erlauben sie (i) die Einfachheit des Einbringens von Teilchen in solche Silicon- oder organische Phasen, wie Dichtmittel, Farben, Beschichtungen, Fetten, Klebstoffen, Antischaummitteln und Gießharzverbindungen, und (ii) stellen modifizierte rheologische, physikalische oder energieabsorbierende Eigenschaften solcher Phasen, entweder in ihrem reinen oder in ihrem Endzustand, bereit.

Zusätzlich sind die Organopolysiloxangele in der Lage, als Träger für Pharmazeutika, Biozide, Herbizide, Pestizide und andere biologische aktive Substanzen zu wirken.

Weiterhin finden die Zusammensetzungen Anwendung als Additive für nichtgewebte Trägersubstrate auf Cellulosebasis oder nichtgewebte synthetische Trägersubstrate, die in feuchten Reinigungstüchern wie Feuchttüchern, feuchten Papiertüchern und feuchten Handtüchern verwendet werden, die im Allgemeinen für Körperhygiene- und Haushaltsreinigungszwecke vermarktet werden.

Die erfindungsgemäßen Organopolysiloxangele können als Träger zur gesteuerten und leicht regulierten Freisetzung einer flüchtigen aktiven organischen Substanz in die freie Atmosphäre verwendet werden, wenn sie mit dieser vermischt werden. Die flüchtige Substanz kann insbesondere ein Parfüm sein oder ein Insektizid oder ein Stoff, der Insekten abwehrt.
In dieser Verwendung finden die erfindungsgemäßen Organopolysiloxangele breite Anwendung, beispielsweise bei der Ausrüstung von Fasern, Textilien und Stoffen aus Baumwolle oder Kunstfasern, Geweben, Tüchern, auch Papiertüchern, Toilettenpapier oder Wischpapier, wie Servietten oder Küchenrolle, oder Vlies, zur langanhaltenden, kontrollierten Beduftung oder Insektenabwehr. Die Mischung aus dem erfindungsgemäßen Organopolysiloxangel und der flüchtigen aktiven organischen Substanz kann auch in Waschmaschinen und Wäschetrockner direkt als solche oder als Zusatz zu Waschmitteln und Weichspülern auf Stoffe und Textilien appliziert werden.

Die Verwendung der erfindungsgemäßen Organopolysiloxangele als Träger zur gesteuerten und leicht regulierten Freisetzung einer flüchtigen aktiven organischen Substanz findet insbesondere Anwendung in den oben genannten kosmetischen Anwendungen, wo sie einen Zusatzeffekt zum dort beschrieben Effekt erzielen können, indem sie beispielsweise ein Parfüm gesteuert abgeben. Die erfindungsgemäßen Organopolysiloxangele können auch in Insektenabwehrpräparaten eingesetzt werden, wo sie ein Insektizid oder einen Stoff, der Insekten abwehrt, abgeben. Solche Produkte können beispielsweise direkt auf die Haut oder auf die Kleidung aufgebracht werden.

In einer weiteren Anwendung können die Mischung aus dem erfindungsgemäßen Organopolysiloxangel und der flüchtigen aktiven organischen Substanz zur kontrollierten Beduftung oder Insektenabwehr in geschlossenen Räumen, wie zum Beispiel in Wohnräumen, Geschäftsräumen, WCs oder Fahrzeugen, wie Busse und Autos, eingesetzt werden.

### Beispiele:

Die nachfolgenden Beispiele dienen dazu, die Erfindung weiter zu erläutern und ihre Funktion und Anwendung in der Praxis zu beschreiben. Sie sind in diesem Sinne illustrierend zu verstehen, nicht beschränkend.

In den Beispielen werden physikalische Größen angegeben, die nach den nachfolgend beschriebenen Messmethoden bestimmt wurden. Falls Angaben zur genauen Nachvollziehbarkeit eines Messwertes im Beispieltext fehlen, sind diese in den hier nachfolgenden Beschreibungen der Messmethoden bereits angegeben. D.h. in diesem Fall gelten als weitere Angaben die in den Texten zu den Messmethoden angegebenen.

### Analytische Methoden:

Die Viskosität der Organopolysiloxangele wurden nach DIN EN ISO 3219 bei Schergeschwindigkeit 1/s und 25°C bestimmt.

Die Viskosität der Organopolysiloxane, wie Si-H-haltige Vernetzer, Organopolysiloxanharze und Polydimethylsiloxane, wurden nach DIN 53019 im linearen Bereich bei 25°C bestimmt.

Die Jodzahl wurde nach DIN 53241-1 gemäß dem Verfahren nach Wijs bestimmt.

Gelpermeationschromatografie zur Bestimmung des gewichtsmittleren Molekulargewichts Mw wurde durchgeführt nach ISO 16014-1 und ISO 16014-3.

Die erfindungsgegenständlichen Organopolysiloxangele bewirken in kosmetischen Anwendungen sensorische Vorteile, indem sie die Verteilbarkeit des Produkts auf der Haut verbessern und dem Produkt ein seidig sanftes Gefühl verleihen. Vergleichbar sind die Organopolysiloxangele dabei in ihrer Leistungsfähigkeit nur dann, wenn sie für die sensorische Prüfung auf eine einheitliche Viskosität eingestellt werden. Als besonders vorteilhaft hat sich hierfür eine Viskosität im Bereich von 75000 - 120000 mPas bei 25°C erwiesen. Ein Kriterium für die erfolgreiche Herstellung der Organopolysiloxangele ist daher die Möglichkeit, diesen Viskositätskorridor einzustellen. Ist dies nicht möglich, ist die Vergleichbarkeit mit den anderen Organopolysiloxangelen nicht gegeben. Es ist ein besonderes Kennzeichen des erfindungsgemäßen Verfahrens, dass es die Einstellung einer Zielviskosität, wie hier 75000 - 120000 mPas verfahrensunabhängig unter Erhalt der sensorischen Eigenschaften gestattet. Dies wird in den nachfolgenden Beispielen belegt und diese Eigenschaft gegen den übrigen Stand der Technik abgegrenzt.

Die Beurteilung der sensorischen Eigenschaften der nachfolgend in den Beispielen beschriebenen Organopolysiloxangele erfolgte durch eine geschulte Gruppe von 5 Probanden (Panelisten).

Die Panelisten haben auf den gereinigten Unterarm auf einer kreisrunden Fläche von 20 cm² jeweils 0,05 g des Produktes aufgetragen und die Organopolysiloxangele im Hinblick auf ihre Verteilbarkeit relativ zueinander verglichen. Das Auftragen erfolgte mit dem Zeigefinger oder Mittelfinger und einer Rotationsgeschwindigkeit von zwei Umdrehungen pro Sekunde. Insgesamt wurden 30 Umdrehungen durchgeführt. Nach einer Wartezeit von 60 Sekunden wurden die Rückstände der Organopolysiloxangele im Hinblick auf ihre Seidigkeit relativ zueinander verglichen.

Relativ zueinander direkt vergleichbar sind dabei stets nur die Organopolysiloxangele, die unter Verwendung des gleichen oder zumindest eines gleichartigen Verdünnungsmittels, also insbesondere mit einem flüchtigen oder einem nicht flüchtigen, hergestellt wurden. Da durch unterschiedliche Verdünnungsmittel ein unterschiedliches Verhalten beim Auftragen und beim Rückstand resultiert, müssen Produkte mit unterschiedlichen Verdünnungsmitteln jeweils getrennt voneinander bewertet werden.

### Beispiel 1 (erfindungsgemäß):

Ein 2000 ml-Reaktionsgefäß aus Glas wird mit einem Kühler mit aufgesetzter Stickstoffeinleitung, Heizmantel, Ankerrührer und Temperaturregelung ausgestattet. In das Reaktionsgefäß werden 497 g eines linearen trimethylsilylterminierten Polydimethylsiloxanes mit einer Viskosität von 5 mPas bei 25°C gegeben.

Danach werden 82 g des kurzkettigen Si-H-haltigen Polydimethylsiloxans der Nummer 14 aus Tabelle 1 (Kettenlänge 140) und 20 g des langkettigen Si-H-haltigen Polydimethylsiloxans der Nummer 28 aus Tabelle 2 (Kettenlänge 450) zugegeben.
Dann fügt man 109,55 g einer 50%-igen Lösung eines MQ-Harzes (M / M^{Vi} / Q = 7,6 / 1 / 11,4; Mₙ = 2570, M_{w} = 5440, Jodzahl=18; M = Me₃SiO_{1/2}, M^{Vi} = Me₂ViSiO_{1/2}, Q = SiO_{4/2} mit Me = Methylrest und Vi= Vinylrest) in dem gleichen linearen trimethylsilylterminierten Polydimethylsiloxan mit einer Viskosität von 5 mPas bei 25°C, das auch als Verdünnungsmittel verwendet wird, hinzu. Zuletzt gibt man 0,7 g einer Mischung von Platin-1,3-Divinyl-1,1,3,3-tetramethyldisiloxan-Komplex in Divinyltetramethyldisiloxan hinzu, wobei die Mischung so eingestellt ist, dass die zugesetzte Menge 52 Gew.-ppm Pt entspricht, bezogen auf die Summe der Masse aus dem MQ-Harz und den beiden Si-H-haltigen Organopolysiloxanen. Das Reaktionsgefäß wird geschlossen und 5 min mit Stickstoff gespült.

Anschließend wird die Reaktionsmischung bei einer Rührgeschwindigkeit von etwa 200 U/min auf 95°C erhitzt, wobei eine Heizrate von 45°C/h angewendet wird. Die Gelbildung erfolgt innerhalb von 30 Minuten nachdem die Innentemperatur von 95°C erreicht ist. Nach erfolgter Gelbildung wird die Heizung abgeschaltet und weitere 60 Minuten gerührt.

Danach werden 5,83 g Polydimethylsiloxan mit 3-Mercaptopropylgruppen und einer Viskosität von 190 mPas bei 25°C und einem Mercaptangehalt (SH-Gehalt) von 0,29 Gew.-% als Stopper hinzugegeben. Die Menge Mercaptan, die auf diese Weise zugesetzt wurde, entspricht dabei 108 Gew.-ppm, bezogen auf die gesamte Menge an Si-H-haltigen Organopolysiloxanen und MQ-Harz, und ist damit mehr als doppelt so groß wie die verwendete Menge Platin.

Man fügt des Weiteren 89,68 g des als Verdünnungsmittels eingesetzten Polydimethylsiloxans mit einer Viskosität von 5 mPas bei 25°C zu und rührt den Stopper und das weitere Verdünnungsmittel unter Schwenken 5 Minuten lang mit einem ULTRA-TURRAX® T 50 bei 6000 U/min ein. Man wiederholt diesen Vorgang noch zweimal mit jeweils der gleichen Menge Verdünnungsmittel. Es wird dabei kein weiterer Stopper hinzugefügt. Auf diesem Weg erhält man ein cremiges, transparentes Gel mit sehr glatter Konsistenz, das zur Verwendung in Kosmetikprodukten geeignet ist.

Der Festgehalt, also der gesamte Gehalt an Netzwerk aus MQ-Harz und den beiden Si-H-haltigen Organopolysiloxanen, sowie dem Stopper und dem Katalysator im Verdünnungsmittel, beträgt nach Verdünnen 16 Gew.-%.
Das erhaltene Organopolysiloxangel besitzt eine Viskosität von 117 000 mPas bei 25°C.

Nach der Bewertung der Panelisten ist das erhaltene Organopolysiloxangel sehr gut verteilbar. Der Rückstand wurde als reichhaltig und mehrheitlich als überwiegend seidig und samtig eingestuft.

### Beispiel 2 (erfindungsgemäß):

Die Vorgehensweise entspricht der in Beispiel 1 beschriebenen, mit dem Unterschied, dass in Beispiel 2 statt des nicht flüchtigen linearen Verdünnungsmittels als Verdünnungsmittel das flüchtige Decamethylpentacyclosiloxan eingesetzt wird. Nach Verdünnen auf einen Festgehalt von 16 Gew.-% beträgt die Viskosität 152 000 mPas bei 25°C.

Nach der Bewertung der Panelisten ist das erhaltene Organopolysiloxangel sehr gut verteilbar. Der Rückstand wurde als reichhaltig und mehrheitlich als trocken, seidig und samtig eingestuft.

### Beispiel 3 (erfindungsgemäß):

Die Vorgehensweise entspricht im Wesentlichen der in Beispiel 1 beschriebenen. Im Unterschied zu Beispiel 1 wird als Verdünnungsmittel anstelle eines nicht flüchtigen trimethylsilylterminierten linearen Polydimethylsiloxans mit einer Viskosität von 5 mPas bei 25°C ein flüchtiges trimethylsilylterminiertes lineares Polydimethylsiloxan mit einer Viskosität von 2 mPas bei 25°C eingesetzt. Die ins Reaktionsgefäß vorgelegte Menge an Verdünnungsmittel beträgt 425,0 g. Anstelle des kurzkettigen Si-H-haltigen Polydimethylsiloxans mit der Nummer 14 aus Tabelle 1 werden 67,75 g des kurzkettigen Si-H-haltigen Polydimethylsiloxans mit der Nummer 11 aus Tabelle 1 (Kettenlänge 75) eingewogen und 20,0 g des langkettigen Si-H-haltigen Polydimethylsiloxans mit der Nummer 28 aus Tabelle 2 (Kettenlänge 450) zugegeben. Es werden 100,0 g der gleichen MQ-Harzzubereitung, wie in Beispiel 1 verwendet, zugegeben. Zuletzt werden 3,0 g einer Mischung von Platin-1,3-Divinyl-1,1,3,3-tetramethyldisiloxan-Komplex in Divinyltetramethyldisiloxan verdünnt mit dem trimethylsilylterminierten Polydimethylsiloxan mit Viskosität von 2 mPas bei 25°C zugegeben, wobei die Mischung so eingestellt ist, dass die zugesetzte Menge 26 Gew.-ppm Pt entspricht, bezogen auf die Summe der Masse aus dem MQ-Harz und den beiden Si-H-haltigen Polydimethylsiloxanen.

Die weitere Vorgehensweise entspricht der in Beispiel 1 beschriebenen. Zum Stoppern werden 2,0 g Polysiloxan mit 3-Mercaptopropylgruppen und einer Viskosität von 190 mPas bei 25 °C und einem Mercaptangehalt (SH-Gehalt) von 0,29 Gew.-% als Stopper hinzugegeben. Die Menge Mercaptan, die auf diese Weise zugesetzt wurde, entspricht dabei 42 Gew.-ppm, bezogen auf die gesamte Menge an Si-H-haltigen Polydimethylsiloxanen und MQ-Harz, und entspricht damit der 1,6-fachen der verwendeten Menge an Platin.

Zum Verdünnen dieses Basisgels werden nun im ersten Verdünnungsschritt 57,75 g weiteres Verdünnungsmittel zugegeben, im zweiten Schritt 67,54 g und im dritten Schritt 104,3 g. Die Vorgehensweise bei der Verdünnung entspricht der gleichen wie in Beispiel 1 beschrieben.

Der finale Festgehalt, d.h. der Gehalt an Netzwerk aus den Si-H-haltigen Polydimethylsiloxanen und dem MQ-Harz zusammen mit der Menge eingesetztem Katalysator und dem Stopperöl liegt bei 16 Gew.-%. Folgende Viskositäten wurden während des Verdünnens gemessen:
Vor der ersten Verdünnung: Festgehalt 22,5%: 344 000 mPas
Nach der ersten Verdünnung: Festgehalt: 20,5%: 258 000 mPas
Nach der zweiten Verdünnung: Festgehalt: 18,5%: 169 000 mPas
Nach der dritten Verdünnung: Festgehalt: 16%: 91 400 mPas

Nach der Bewertung der Panelisten ist das erhaltene Organopolysiloxangel sehr gut verteilbar. Der Rückstand wurde als reichhaltig und mehrheitlich als trocken, seidig und samtig eingestuft.

### Beispiel 4 (erfindungsgemäß, in einem zweistufigen Prozess):

Die Vorgehensweise entspricht derjenigen gemäß Beispiel 1, wobei hier die Hydrosilylierung zweistufig gefahren wird. Zuerst wird das kurzkettige Si-H-haltige Polydimethylsiloxan alleine hydrosilyliert, danach wird das langkettige Si-H-haltige Polydimethylsiloxan hydrosilyliert. Als Lösemittel wird hier anstelle des nicht flüchtigen trimethylsilylterminierten Polydimethylsiloxans mit einer Viskosität von 5 mPas aus Beispiel 1 Decamethylcyclopentasiloxan eingesetzt. Außerdem wird in diesem Beispiel nicht nachträglich verdünnt, sondern die gesamte erforderliche Verdünnungsmittelmenge von Anfang an zugesetzt.

Ein 2000 ml-Reaktionsgefäß aus Glas wird mit einem Kühler mit aufgesetzter Stickstoffeinleitung, Heizmantel, Ankerrührer und Temperaturregelung ausgestattet. In das Reaktionsgefäß werden 446,4 g Decamethylcyclopentasiloxan gegeben. Danach werden 62,5 g einer 50%-igen Lösung eines MQ-Harzes (M / M^{Vi} / Q = 7,6 / 1 / 11,4, Mₙ = 2570, M_{w} = 5440, Jodzahl = 18), wie in Beispiel 1 beschrieben, in einem nicht flüchtigen linearen trimethylsilylterminierten Polydimethylsiloxan mit einer Viskosität von 5 mPas bei 25°C zugesetzt, gefolgt von 46,8 g des kurzkettigen Si-H-haltigen Polydimethylsiloxans der Nummer 14 aus Tabelle 1 (Kettenlänge 140). Zuletzt gibt man 0,4 g einer Mischung von Platin-1,3-Divinyl-1,1,3,3-tetramethyldisiloxan-Komplex in Divinyltetramethyldisiloxan hinzu, wobei die Mischung so eingestellt ist, dass die zugesetzte Menge 51 Gew.-ppm Pt entspricht, bezogen auf die Summe der Masse aus dem MQ-Harz und den beiden Si-H-haltigen Polydimethylsiloxanen. Zunächst wurde hier nur das kurzkettige Si-H-haltige Polydimethylsiloxan zugesetzt, die Menge Platin ist allerdings bereits auf die Summe aus der Menge beider Si-H-haltigen Polydimethylsiloxane berechnet. Das Reaktionsgefäß wird geschlossen und 5 min mit Stickstoff gespült.

Anschließend wird die Reaktionsmischung bei einer Rührgeschwindigkeit von etwa 200 U/min auf 95°C erhitzt, wobei eine Heizrate von 45°C/h angewendet wird. Die Gelbildung erfolgt innerhalb von 30 Minuten nachdem die Innentemperatur von 95°C erreicht ist. Nach erfolgter Gelbildung wird eine Stunde lang weiter bei 95°C Innentemperatur gerührt.

Dann werden 11,7 g des langkettigen Si-H-haltigen Polydimethylsiloxans der Nummer 28 aus Tabelle 2 (Kettenlänge 450) zudosiert und eine weitere Stunde bei 95°C gerührt. Nach dieser Zeit ist die Gelbildung abgeschlossen.

Danach werden 3,24 g Polysiloxan mit 3-Mercaptopropylgruppen und einer Viskosität von 190 mPas bei 25 °C und einem Mercaptangehalt (SH-Gehalt) von 0,29 Gew.-% als Stopper hinzugegeben. Die Menge Mercaptan, die auf diese Weise zugesetzt wurde, entspricht dabei 104 Gew.-ppm, bezogen auf die gesamte Menge an Si-H-haltigen Polydimethylsiloxanen und MQ-Harz, und ist damit doppelt so groß wie die verwendete Menge Platin.

Das erhaltene Gel hat einen Festgehalt von 16 Gew.-% und besitzt eine Viskosität von 98 000 mPas bei 25°C.

Nach der Bewertung der Panelisten ist das erhaltene Organopolysiloxangel sehr gut verteilbar. Der Rückstand wurde als reichhaltig und mehrheitlich als seidig und samtig eingestuft.

### Vergleichsbeispiel 1

### (nicht erfindungsgemäß, nur kurzkettiges Si-H-haltiges Organopolysiloxan, hohe Heizrate):

Die Vorgehensweise entspricht im Wesentlichen der in Beispiel 1 beschriebenen:
Im Unterschied zu Beispiel 1 wird nur ein Si-H-haltiges Polydimethylsiloxan eingesetzt, bei dem es sich um das kurzkettige Si-H-haltige Polydimethylsiloxan der Nummer 14 aus Tabelle 1 (Kettenlänge 140, a:b = 50:1) handelt. Davon werden 102,4 g eingesetzt.
Die Heizrate wird in diesem Beispiel auf 90°C/h eingestellt. Eine derart hohe Heizrate ist im Labor in diesem Maßstab leicht zu realisieren, betrieblich jedoch bei einer Größe von 1 m³ oder mehr nicht mehr möglich, selbst wenn man mit Dampf mit hohem Druck arbeitet. Zudem besteht die Gefahr starker lokaler Überhitzung, die zum Austrocknen des Gels an diesen Stellen und irreversibler Partikelbildung führt, die die sensorischen Eigenschaften des Gels zunichtemacht.
Man erhält ein Gel mit einem Festgehalt von 16 Gew.-% und einer Viskosität von 108 000 mPas bei 25°C.

Nach der Bewertung der Panelisten ist das erhaltene Organopolysiloxangel sehr gut verteilbar. Der Rückstand wurde als reichhaltig und mehrheitlich als überwiegend seidig und samtig eingestuft.

### Vergleichsbeispiel 2

### (nicht erfindungsgemäß, analog Vergleichsbeispiel 1, aber mit niedriger Heizrate):

Die Vorgehensweise entspricht der von Vergleichsbeispiel 1 mit dem Unterschied, dass nun mit einer Heizrate von 45°C/h aufgeheizt wird. Das Produkt erreicht keine gelartige Konsistenz, sondern bleibt fließfähig und entspricht in seinen Eigenschaften eher einem Öl. Es verläuft bei dem Versuch, es zu prüfen, bereits vor dem manuellen Verteilen auf der Haut von alleine.
Im Gegensatz zu Vergleichsbeispiel 1 hinterlässt es keinen matten, seidigen Film, sondern eine ölige, glänzende Schicht, die von keinem der Panelisten als seidig bewertet worden ist.

Es ist nicht für die Anwendung in kosmetischen Produkten geeignet, da es keine geeignete Konsistenz und keine geeigneten sensorischen Eigenschaften aufweist.

Das Verfahren in der Zusammensetzung gemäß Vergleichsbeispiel 1 kann aufgrund seiner Empfindlichkeit gegenüber der Heizrate in der gegebenen Zusammensetzung nicht vom Labormaßstab (mit einer hohen Heizrate) in den Produktionsmaßstab von 1m³ oder größer (mit einer niedrigen Heizrate) vergrößert werden.

### Vergleichsbeispiel 3

### (nicht erfindungsgemäß, analog Vergleichsbeispiel 2 mit niedriger Heizrate aber längerkettigem Si-H-haltigem Organopolysiloxan):

Die Vorgehensweise entspricht der in Vergleichsbeispiel 2 beschriebenen, mit dem Unterschied, dass als Si-H-haltiges Organopolysiloxan das langkettige Si-H-haltige Polydimethylsiloxan der Nummer 28 aus Tabelle 2 (Kettenlänge 450, a:b = 50:1) verwendet wird anstelle des kurzkettigen. Es wird nur dieses eine Si-H-haltige Polydimethylsiloxan verwendet.

Man erhält ein Elastomergel, das bei einem Festgehalt von 16 Gew.-% eine Viskosität von 205 000 mPas bei 25°C aufweist und das bei einem Festgehalt von 14 Gew.-% eine Viskosität von 122 000 mPas bei 25°C aufweist.
Im Unterschied zu Vergleichsbeispiel 1 wird bei einem gewünschten Festgehalt von 16 Gew.-% eine viel höhere Viskosität erhalten. Wird der Festgehalt auf 14 Gew.-% verringert, wird erst dann eine vergleichbare Viskosität wie beim Vergleichsbeispiel 1 erhalten. Beides, der Festgehalt und die Viskosität, beeinflussen aber die sensorischen Eigenschaften des Gels.

Nach der Bewertung des auf 122 000 mPas eingestellten Gels durch die Panelisten ist das erhaltene Organopolysiloxangel sehr gut verteilbar. Der Rückstand wurde als reichhaltig und als seidig und ölig eingestuft.

Das erhaltene Gel ist zur Herstellung kosmetischer Produkte geeignet, jedoch von dem entsprechenden Gel aus Vergleichsbeispiel 1 sensorisch gut zu unterscheiden. Im direkten Vergleich bevorzugten 4 von 5 Panelisten das Gel aus Vergleichsbeispiel 1 gegenüber diesem Gel.

Der Versuch wurde anschließend mit den gleichen Panelisten wiederholt, wobei ihnen nicht mitgeteilt wurde, dass sie erneut die gleichen Gele miteinander vergleichen. Bei der Wiederholung bevorzugten 5 von 5 Panelisten das Gel aus Vergleichsbeispiel 1 gegenüber diesem Gel.
Bei der Übertragung vom Labormaßstab mit hoher Heizrate auf den Produktionsmaßstab mit niedriger Heizrate kann bei Ersatz des kurzkettigen Si-H-haltigen Organopolysiloxanes durch das langkettige Si-H-haltige Organopolysiloxan kein Organopolysiloxangel mit vergleichbaren sensorischen Eigenschaften erzeugt werden.

### Beispiel 5

### (erfindungsgemäß, Anpassung der Mengen an Si-H-haltigen Organopolysiloxanen an veränderte Heizrate unter Erhalt der gleichen Eigenschaften des Organopolysiloxangels):

Die Vorgehensweise entspricht der in Beispiel 1 beschriebenen. Im Unterschied zu Beispiel 1 wird nun allerdings nicht mit 45°C/h geheizt, sondern mit 90°C/h, also eine doppelt so hohe Heizrate angewendet.
Im Unterschied zu Beispiel 1 werden nun folgende Mengen der Si-H-haltigen Polydimethylsiloxane eingesetzt:
92 g des Si-H-haltigen Polydimethylsiloxans der Nummer 14 aus Tabelle 1 (Kettenlänge 140) und 10 g des Si-H-haltigen Polydimethylsiloxans der Nummer 28 aus Tabelle 2 (Kettenlänge 450, a:b = 50:1).
Ansonsten entspricht die Versuchsdurchführung exakt der in Beispiel 1 beschriebenen. Als Ergebnis wird ein Organopolysiloxangel mit einer Viskosität von 121 000 mPas erhalten bei einem Festgehalt von 16 Gew.-%.

Nach der Bewertung der Panelisten ist das erhaltene Organopolysiloxangel sehr gut verteilbar. Der Rückstand wurde als reichhaltig und mehrheitlich als überwiegend seidig und samtig eingestuft.

Im direkten Vergleichsversuch bevorzugten 3 von 5 Panelisten das Gel aus Beispiel 1 gegenüber diesem Gel aus Beispiel 5. Der Vergleichsversuch wurde mit den gleichen Panelisten wiederholt, wobei ihnen nicht mitgeteilt wurde, dass sie erneut die gleichen Gele miteinander vergleichen. Bei der Wiederholung bevorzugten 2 von 5 Panelisten das Gel aus Beispiel 1 gegenüber diesem Gel aus Beispiel 5, wobei nur einer der zwei Panelisten, die das Gel aus Beispiel 1 im Wiederholungsversuch bevorzugten, in ersten Versuch bereits zu diesem Ergebnis gekommen ist. Eine sichere Unterscheidbarkeit der beiden Gele aus Beispiel 1 und aus Beispiel 5 aufgrund ihrer sensorischen Eigenschaften ist daher nicht gegeben.

Dies bedeutet, dass die beiden Gele praktisch gleichwertige Eigenschaften aufweisen und der erfindungsgemäße Einsatz von zwei Arten von Si-H-haltigen Organopolysiloxanen, einem kurzkettigen und einem langkettigen, die Anpassung an veränderte Rahmenbedingungen, wie Änderung der Heizrate, erlaubt unter Erhalt der gewünschten Eigenschaften des Organopolysiloxangels, was bei Verwendung von nur einer Art von Si-H-haltigem Organopolysiloxan, nur einem kurzkettigen oder nur einem langkettigen, nicht möglich wäre.

## Patentansprüche

1. Verfahren zur Herstellung von Organopolysiloxangelen indem
(1a) ungesättigte Organopolysiloxanharze aus Einheiten der Formeln
SiO₂
(Q-Einheiten) und
R₃SiO_{1/2}
und
R₂R¹SiO_{1/2}
(M-Einheiten), wobei
R gleich oder verschieden sein kann und einen einwertigen, gegebenenfalls substituierten Kohlenwasserstoffrest mit 1 bis 18 Kohlenstoffatomen je Rest bedeutet,
R¹ einen einwertigen Kohlenwasserstoffrest bedeutet, an den Si-H-Gruppen in einer Hydrosilylierungsreaktion angelagert werden können, vorzugsweise ein ω-Alkenylrest mit 2 bis 12 C-Atomen, bevorzugt ein Vinylrest ist,
mit der Maßgabe, dass die Organopolysiloxanharze mindestens 2 Reste R¹, vorzugsweise mindestens 3 Reste R¹, enthalten und dass das molare Verhältnis von M-Einheiten zu Q-Einheiten im Bereich von 0,5 bis 4,0, vorzugsweise im Bereich von 0,5 bis 2,0, liegt, die Organopolysiloxanharze neben den M- und Q-Einheiten noch geringe Mengen an RSiO_{3/2} (T)-Einheiten oder R₂SiO_{2/2} (D)-Einheiten, in Mengen von 0,01 bis 20 Mol%, bezogen auf die Summe aller Siloxaneinheiten, enthalten können und die Organopolysiloxanharze bis zu 10 Gew.-% freie Si-gebundene Hydroxy- oder Alkoxygruppen enthalten können,
und gegebenenfalls
(1b) Verbindungen, die eine polare organische Gruppe, vorzugsweise Glycosidgruppe, wie Oligo- oder Polysaccharidgruppe, Polyoxyalkylengruppe, wie Polyoxyethylen- oder Polyoxypropylenguppe, Hydroxy-, Amid- oder Carboxygruppe und eine hydrosilylierbare Endgruppe aufweisen, mit
(2) einem Gemisch aus zwei Si-H-haltigen Organopolysiloxanen, die unterschiedliche mittlere Kettenlängen aufweisen, der Formel
(R²₃₋ₓHₓSiO_{1/2})(R²₂SiO_{2/2})ₐ(R²HSiO_{2/2})_{b}(R²₃₋ₓHₓSiO_{1/2}) (I),
wobei R² gleich oder verschieden ist und einen unsubstituierten oder gegebenenfalls mit Heteroatomen substituierten, aliphatischen, cycloaliphatischen oder aromatischen, ggf. polycyclischen, C₁-C₁₈-Kohlenwasserstoffrest bedeutet,
x 0 oder 1 ist,
a und b jeweils ganze Zahlen ≥ 0 sind,
mit der Maßgabe, dass die Summe a+b ≥ 30 ist,
dass die Organopolysiloxane durchschnittlich mindestens 2 Si-gebundene H-Atome, vorzugsweise mindestens 3 Si-gebundene H-Atome, enthalten und dass das längerkettige der beiden Organopolysiloxane mindestens die 3-fache Kettenlänge (a+b) des kurzkettigen Organopolysiloxans aufweist,
in Gegenwart von
(3) die Anlagerung von Si-gebundenem Wasserstoff an aliphatische Mehrfachbindungen fördernde Katalysatoren,
umgesetzt werden,
wobei (1a), ggf. (1b) und (2) in
(4) Verdünnungsmitteln, vorzugsweise Organopolysiloxanen mit 2 bis 200 Si-Atomen, bevorzugt 2 bis 50 Si-Atomen, oder organischen Verdünnungsmitteln oder Mischungen von Organopolysiloxanen mit 2 bis 200 Si-Atomen, bevorzugt 2 bis 50 Si-Atomen, und organischen Verdünnungsmitteln, dispergiert sind
und die Umsetzungsreaktion durch Zugabe von
(5) als Katalysatorgifte eingesetzte Stopperverbindungen gestoppt wird.

2. Verfahren nach Anspruch 1, **dadurch gekennzeichnet, dass** in der Formel (I) x gleich 0 ist.

3. Verfahren nach Anspruch 1 oder 2, **dadurch gekennzeichnet, dass** in der Formel (I) die Summe a+b ≥ 55 ist.

4. Verfahren nach Anspruch 1, 2 oder 3, **dadurch gekennzeichnet, dass** als Katalysatoren (3) Metallkatalysatoren, vorzugsweise Platinkatalysatoren, in Mengen von 1 bis 100 Gew.-ppm (Gewichtsteilen je Million Gewichtsteilen), berechnet als elementares Metall, vorzugsweise elementares Platin, jeweils bezogen auf das Gesamtgewicht der Komponenten (1a), ggf. (1b) und (2), eingesetzt werden.

5. Verfahren nach einem der Ansprüche 1 bis 4, **dadurch gekennzeichnet, dass** als Verdünnungsmittel (4) Polydimethylsiloxane mit 2 bis 50 Si-Atomen, aliphatische oder alicyclische Kohlenwasserstoffe mit 4 bis 30 C-Atomen oder Ester von Carbonsäuren mit 2 bis 30 C-Atomen eingesetzt werden.

6. Verfahren nach einem der Ansprüche 1 bis 5, **dadurch gekennzeichnet, dass** als Stopperverbindungen (5) Verbindungen eingesetzt werden, die als Katalysatorgifte wirkende funktionelle Gruppen aufweisen, vorzugsweise als Stopperverbindungen (5) Mercaptogruppen (SH) aufweisende organische Verbindungen oder Mercaptoalkylgruppen aufweisende Organopolysiloxane, bevorzugt 3-Mercaptopropylgruppen aufweisende Organopolysiloxane, eingesetzt werden.

7. Verfahren nach einem der Ansprüche 1 bis 6, **dadurch gekennzeichnet, dass** die Stopperverbindungen (5) in Mengen von mindestens 1,1 Mol die Stopperung bewirkende funktionelle Gruppe, vorzugsweise Mercaptogruppe, je Mol elementares Metall, insbesondere elementares Platin, im Katalysator (3) eingesetzt werden.

8. Verfahren nach einem der Ansprüche 1 bis 7, **dadurch gekennzeichnet, dass** die nach der Umsetzung erhaltenen Organopolysiloxangele homogenisiert werden, wobei lagerstabile Organopolysiloxangele erhalten werden, wobei lagerstabil bedeutet, dass sich die gebildeten Organopolysiloxangele innerhalb von 6 Monaten Lagerung bei Raumtemperatur (20°C) nicht in zwei oder mehr Phasen entmischen.

9. Verfahren nach einem der Ansprüche 1 bis 8, **dadurch gekennzeichnet, dass** die so erhaltenen Organopolysiloxangele mit weiteren Verdünnungsmitteln (4) und/oder aktiven Wirkstoffen für die Körperpflege oder Gesundheitspflege verdünnt werden und ggf. anschließend homogenisiert werden.

10. Verwendung der nach einem der Ansprüche 1 bis 9 hergestellten Organopolysiloxangele in kosmetischen Zusammensetzungen.

## Claims

1. Process for producing organopolysiloxane gels by reacting
(1a) unsaturated organopolysiloxane resins composed of units of the formulae
SiO₂ (Q units) and
R₃SiO_{1/2} and R₂R¹SiO_{1/2} (M units),
where
R may be the same or different and is a monovalent, optionally substituted hydrocarbon radical having 1 to 18 carbon atoms per radical, R¹ is a monovalent hydrocarbon radical onto which Si-H groups may be added in a hydrosilylation reaction, preferably an ω-alkenyl radical having 2 to 12 carbon atoms, preferably a vinyl radical,
with the proviso that the organopolysiloxane resins contain at least 2 R¹ radicals, preferably at least 3 R¹ radicals, and that the molar ratio of M units to Q units is in the range from 0.5 to 4.0, preferably in the range from 0.5 to 2.0, the organopolysiloxane resins may contain, as well as the M and Q units, also small amounts of RSiO_{3/2} (T) units or R₂SiO_{2/2} (D) units, in amounts of 0.01 to 20 mol%, based on the sum total of all siloxane units, and the organopolysiloxane resins may contain up to 10% by weight of free Si-bonded hydroxyl or alkoxy groups,
and optionally
(1b)compounds having a polar organic group, preferably glycoside group, such as oligo- or polysaccharide group, polyoxyalkylene group, such as polyoxyethylene or polyoxypropylene group, hydroxyl, amide or carboxyl group and a hydrosilylatable end group, with
(2) a mixture of two Si-H-containing organopolysiloxanes which have different average chain lengths and are of the formula
(R²₃₋ₓHₓSiO_{1/2})(R²₂SiO_{2/2})ₐ(R²HSiO_{2/2})_{b}(R²₃₋ₓHₓSiO_{1/2}) (I)
where R² is the same or different and is an unsubstituted or optionally heteroatom-substituted, aliphatic, cycloaliphatic or aromatic, optionally polycyclic, C₁-C₁₈ hydrocarbon radical,
x is 0 or 1,
a and b are each integers ≥ 0,
with the proviso that the sum total of a+b ≥ 30, that the organopolysiloxanes contain an average of at least 2 Si-bonded hydrogen atoms, preferably at least 3 Si-bonded hydrogen atoms, and that the long-chain of the two organopolysiloxanes has at least 3 times the chain length (a+b) of the short-chain organopolysiloxane,
in the presence of
(3) catalysts that promote the addition of Si-bonded hydrogen onto aliphatic multiple bonds,
where (1a), optionally (1b) and (2) are dispersed in
(4) diluents, preferably organopolysiloxanes having 2 to 200 silicon atoms, preferably 2 to 50 silicon atoms, or organic diluents or mixtures of organopolysiloxanes having 2 to 200 silicon atoms, preferably 2 to 50 silicon atoms, and organic diluents,
and the reaction is stopped by addition of
(5) stopper compounds used as catalyst poisons.

2. Process according to Claim 1, **characterized in that** x = 0 in the formula (I).

3. Process according to Claim 1 or 2, **characterized in that** the sum of a+b ≥ 55 in the formula (I).

4. Process according to Claim 1, 2 or 3, **characterized in that** catalysts (3) used are metal catalysts, preferably platinum catalysts, in amounts of 1 to 100 ppm by weight (parts per weight per million parts by weight), calculated as elemental metal, preferably elemental platinum, based in each case on the total weight of components (1a), optionally (1b) and (2).

5. Process according to any of Claims 1 to 4, **characterized in that** diluents (4) used are polydimethylsiloxanes having 2 to 50 silicon atoms, aliphatic or alicyclic hydrocarbons having 4 to 30 carbon atoms or esters of carboxylic acids having 2 to 30 carbon atoms.

6. Process according to any of Claims 1 to 5, **characterized in that** stopper compounds (5) used are compounds having functional groups that act as catalyst poisons, and stopper compounds (5) used are preferably organic compounds having mercapto groups (SH) or organopolysiloxanes having mercaptoalkyl groups, preferably organopolysiloxanes having 3-mercaptopropyl groups.

7. Process according to any of Claims 1 to 6, **characterized in that** the stopper compounds (5) are used in amounts of at least 1.1 mol of functional group that brings about the stopping, preferably mercapto group, per mole of elemental metal, especially elemental platinum, in the catalyst (3).

8. Process according to any of Claims 1 to 7, **characterized in that** the organopolysiloxane gels obtained after the reaction are homogenized to obtain storage-stable organopolysiloxane gels, "storage-stable" meaning that the organopolysiloxane gels formed do not separate into two or more phases in the course of storage at room temperature (20°C) for six months.

9. Process according to any of Claims 1 to 8, **characterized in that** the organopolysiloxane gels thus obtained are diluted with further diluents (4) and/or active ingredients for personal care or healthcare and then optionally homogenized.

10. Use of the organopolysiloxane gels produced according to any of Claims 1 to 9 in cosmetic compositions.

## Revendications

1. Procédé pour la préparation de gels d'organopolysiloxane, dans lequel on transforme
(1a) des résines insaturées d'organopolysiloxane constituées par des unités de formule
SiO₂
(unités Q)
et
R₃SiO_{1/2}
et
R₂R¹SiO_{1/2}
(unités M) dans lesquelles
R peut être identique ou différent et signifie un radical hydrocarboné monovalent, le cas échéant substitué, comprenant 1 à 18 atomes de carbone par radical,
R¹ signifie un radical hydrocarboné monovalent, sur lequel des groupes Si-H peuvent être additionnés dans une réaction d'hydrosilylation, de préférence un radical ω-alcényle comprenant 2 à 12 atomes de carbone, de préférence un radical vinyle
sous réserve que les résines d'organopolysiloxane contiennent au moins 2 radicaux R¹, de préférence au moins 3 radicaux R¹, et que le rapport molaire des unités M aux unités Q se situe dans la plage de 0,5 à 4,0, de préférence dans la plage de 0,5 à 2,0, les résines de polyorganosiloxane peuvent contenir, outre les unités M et Q, encore de faibles quantités de RSiO_{3/2} (unités T) ou de R₂SiO_{2/2} (unités D), en des quantités de 0,01 à 20% en mole, par rapport à la somme de toutes les unités siloxane et les résines d'organopolysiloxane peuvent contenir jusqu'à 10% en poids de groupes hydroxy ou alcoxy libres liés à Si,
et le cas échéant
(1b) des composés qui présentent un groupe organique polaire, de préférence un groupe glycoside, tel qu'un groupe oligosaccharide ou polysaccharide, un groupe polyoxyalkylène, tel qu'un groupe polyoxyéthylène ou polyoxypropylène, un groupe hydroxy, amide ou carboxy et un groupe terminal hydrosilylable, avec
(2) un mélange de deux organopolysiloxanes contenant Si-H, qui présentent des longueurs de chaîne moyenne différentes, de formule
(R²₃₋ₓHₓSiO_{1/2})(R²₂SiO_{2/2}) a (R²HSiO_{2/2})_{b}(R²₃₋ₓHₓSiO_{1/2}) (I),
dans laquelle R² est identique ou différent et signifie un radical hydrocarboné en C₁-C₁₈ non substitué ou le cas échéant substitué par des hétéroatomes, aliphatique, cycloaliphatique ou aromatique, le cas échéant polycyclique,
x vaut 0 ou 1,
a et b représentent à chaque fois des nombres entiers ≥ 0,
sous réserve que la somme a+b ≥ 30,
que les organopolysiloxanes contiennent en moyenne au moins 2 atomes d'H liés par Si, de préférence au moins 3 atomes d'H liés par Si, et que l'organopolysiloxane présentant la chaîne la plus longue parmi les deux organopolysiloxanes présente une longueur de chaîne représentant au moins le triple (a+b) de celle de l'organopolysiloxane à courte chaîne,
en présence de
(3) catalyseurs favorisant l'addition d'hydrogène lié
par Si sur des liaisons aliphatiques multiples, (la), le cas échéant (1b), et (2) étant
(4) dispersés dans des diluants, de préférence des organopolysiloxanes présentant 2 à 200 atomes de Si, de préférence 2 à 50 atomes de Si, ou dans des diluants organiques ou dans des mélanges d'organopolysiloxanes présentant 2 à 200 atomes de Si, de préférence 2 à 50 atomes de Si, et de diluants organiques
et la réaction de transformation étant
(5) arrêtée par addition de composés d'arrêt utilisés en tant que poisons de catalyseur.

2. Procédé selon la revendication 1, **caractérisé en ce que**, dans la formule (I), x vaut 0.

3. Procédé selon la revendication 1 ou 2, **caractérisé en ce que**, dans la formule (I), la somme a+b ≥ 55.

4. Procédé selon la revendication 1, 2 ou 3, **caractérisé en ce qu'**on utilise, comme catalyseurs (3), des catalyseurs métalliques, de préférence des catalyseurs à base de platine, en des quantités de 1 à 100 ppm en poids (parties en poids par million de parties en poids), calculées comme métal élémentaire, de préférence comme platine élémentaire, à chaque fois par rapport au poids total des composants (la), le cas échéant (1b) et (2).

5. Procédé selon l'une quelconque des revendications 1 à 4, **caractérisé en ce qu'**on utilise, comme diluants (4), des polydiméthylsiloxanes comprenant 2 à 50 atomes de Si, des hydrocarbures aliphatiques ou alicycliques comprenant 4 à 30 atomes de carbone ou des esters d'acide carboxylique comprenant 2 à 30 atomes de carbone.

6. Procédé selon l'une quelconque des revendications 1 à 5, **caractérisé en ce qu'**on utilise, comme composés d'arrêt (5), des composés qui présentent des groupes fonctionnels agissant comme poisons de catalyseur, de préférence, comme composés d'arrêt (5), des composés organiques présentant des groupes mercapto (SH) ou des organopolysiloxanes présentant des groupes mercaptoalkyle, de préférence des organopolysiloxanes présentant des groupes 3-mercaptopropyle.

7. Procédé selon l'une quelconque des revendications 1 à 6, **caractérisé en ce que** les composés d'arrêt (5) sont utilisés en des quantités d'au moins 1,1 mole de groupe fonctionnel provoquant l'arrêt, de préférence le groupe mercapto, par mole de métal élémentaire, en particulier le platine élémentaire, dans le catalyseur (3).

8. Procédé selon l'une quelconque des revendications 1 à 7, **caractérisé en ce que** les gels d'organopolysiloxane obtenus après la transformation sont homogénéisés, des gels d'organopolysiloxane stables à l'entreposage étant obtenus, stable à l'entreposage signifiant que les gels d'organopolysiloxane formés ne se séparent pas en deux phases ou plus pendant un entreposage de 6 mois à température ambiante (20°C).

9. Procédé selon l'une quelconque des revendications 1 à 8, **caractérisé en ce que** les gels d'organopolysiloxanes ainsi obtenus sont dilués par d'autres diluants (4) et/ou par d'autres principes actifs pour les soins corporels ou les soins de la santé et ensuite, le cas échéant, homogénéisés.

10. Utilisation des gels d'organopolysiloxane préparés selon l'une quelconque des revendications 1 à 9 dans des compositions cosmétiques.
